# EUROPEAN PATENT APPLICATION

(11) **EP 3 528 298 A1**
(43) Date of publication of application: **21.08.2019**
(21) Application number: 16918551.9
(22) Date of filing: 13.10.2016
(51) Int. Cl.: H01L 51/50, H05B 33/02, H05B 33/10

(54) **ORGANIC ELECTRONICS MATERIAL, INK COMPOSITION, ORGANIC ELECTRONICS ELEMENT, AND ORGANIC ELECTRONICS ELEMENT PRODUCTION METHOD**

(71) Applicant: Hitachi Chemical Company, Ltd., Chiyoda-ku Tokyo 100-6606 (JP)
(72) Inventor: SUGIOKA, Tomotsugu, Tokyo 100-6606 (JP); ISHITSUKA, Kenichi, Tokyo 100-6606 (JP); YOSHINARI, Yuki, Tokyo 100-6606 (JP); RYUZAKI, Daisuke, Tokyo 100-6606 (JP); ASANO, Naoki, Tokyo 100-6606 (JP)
(74) Representative: Adam, Holger
(86) International application number: PCT/JP2016/080383
(87) International publication number: WO 2018/070019

(57) **Abstract**

The present invention relates to an organic electronic material containing at least an ionic compound represented by general formula (1) shown below and a compound having a charge transport unit. The present invention can provide an organic electronic material that is capable of forming an organic electronic element having a low drive voltage and excellent emission efficiency and lifespan characteristics. In general formula (1), ArF represents a fluoroaryl group or a fluoroheteroaryl group, each of R^{a} and R^{b} independently represents a hydrogen atom (H), an alkyl group, a benzyl group, an aryl group or a heteroaryl group, and A represents an anion.

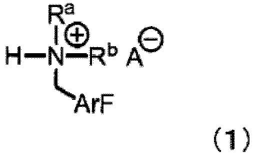

## Description

### TECHNICAL FIELD

The present invention relates to an organic electronic material, and also relates to an ink composition, an organic electronic element and an organic electroluminescent element that use the organic electronic material, and a method for producing an organic electronic element.

### BACKGROUND ART

Organic electronic elements are elements that use an organic substance to perform an electrical operation. Organic electronic elements are expected to be capable of providing advantages such as low energy consumption, low prices and superior flexibility, and are attracting considerable attention as a potential alternative technology to conventional inorganic semiconductors containing mainly silicon.

Examples of organic electronic elements include organic electroluminescent elements (hereafter sometimes referred to as organic EL elements), organic photoelectric conversion elements, and organic transistors and the like.

Among organic electronic elements, organic EL elements are attracting attention for potential use in large-surface area solid state lighting applications to replace incandescent lamps and gas-filled lamps and the like. Further, organic EL elements are also attracting attention as the leading selfluminous display for replacing liquid crystal displays (LCD) in the field of flat panel displays (FPD), and commercial products are becoming increasingly available.

In the field of organic EL elements, tests are being conducted into the use of mixtures of charge transport compounds and electron-accepting compounds, both for the purpose of improving the emission efficiency and the lifespan characteristics, and for the purpose of reducing the drive voltage.

In this type of technique, it is thought that when the charge transport compound and the electron-accepting compound are mixed, a compound composed of a radical cation of the charge transport compound and a counter anion is produced, thereby improving the emission efficiency, the lifespan characteristics and the drive voltage.

For example, Patent Document 1 discloses a composition composed of an ionic compound represented by a prescribed formula and a charge transport compound as a composition for forming a charge transport film.

On the other hand, depending on the materials used and the film production method, organic EL elements are broadly classified into two types: low-molecular weight type organic EL elements and polymer type organic EL elements. In polymer type organic EL elements, the organic material is composed of a polymer material, and compared with low-molecular weight type organic EL elements which require that film formation is conducted in a vacuum system, polymer type organic EL elements can be produced using simple film formation processes such as printing or inkjet processes, and are therefore expected to be indispensable elements in future large-screen organic EL displays.

Both low-molecular weight type organic EL elements and polymer type organic EL elements have already been the subject of much research, but low emission efficiency and short emission lifespan remain significant problems. One technique for addressing these problems involves multilayering in low-molecular weight type organic EL elements.

In low-molecular weight type organic EL elements, because film formation is performed by vapor deposition methods, multilayering can be achieved easily by sequentially changing the compound being used while the vapor deposition is performed. On the other hand, in polymer type organic EL elements, film formation is often performed using wet processes such as printing or inkjet application. When multilayering is performed using wet processes, a problem arises in that the lower layer may dissolve during application of the upper layer. Accordingly, multilayering of polymer type organic EL elements is more difficult than multilayering of low-molecular weight type organic EL elements, and achieving improvements in the emission efficiency and the lifespan characteristics has proven problematic.

Various methods have already been proposed to address this problem. One such method utilizes a difference in the degree of solubility. One example is an element having a two-layer structure composed of a hole injection layer formed from water-soluble polythiophene:polystyrene sulfonate (PEDOT:PSS), and a light-emitting layer formed using an aromatic organic solvent such as toluene. In this case, the PEDOT:PSS layer does not dissolve in the aromatic solvent such as toluene, enabling production of a two-layer structure.

Further, in order to address the multilayering problem outlined above, Patent Document 2 discloses a method that uses the polymerization reaction of a siloxane compound or an oxetanyl group or vinyl group or the like to change the degree of solubility of a compound, thereby making the resulting thin film insoluble in solvents.

As described above, in the field of organic electronic elements, many investigations have been undertaken with the aim of improving various properties of the element such as the drive voltage, the emission efficiency and the lifespan characteristics, but totally satisfactory elements have yet to be produced, and further improvements would be desirable.

### PRIOR ART DOCUMENTS

### PATENT DOCUMENTS

Patent Document 1: JP 2006-233162 A
Patent Document 2: WO 2008/010487

### SUMMARY OF THE INVENTION

### PROBLEMS TO BE SOLVED BY THE INVENTION

The present invention has been developed in light of the above issues, and has an object of providing an organic electronic material that is capable of forming an organic electronic element having a low drive voltage and excellent emission efficiency and lifespan characteristics. Further, the present invention also has the objects of providing an ink composition and an organic electronic element that use the organic electronic material, and a method for producing the organic electronic element.

### MEANS TO SOLVE THE PROBLEMS

As a result of intensive investigation, the inventors of the present invention discovered that an organic electronic material containing a combination of an ionic compound having a specific structure and a compound having a charge transport unit was able to address the problems outlined above, and they were therefore able to complete the present invention.
[1] One aspect of the present invention relates to an organic electronic material containing at least an ionic compound represented by general formula (1) shown below, and a compound having a charge transport unit. 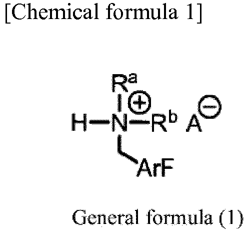 (In general formula (1):
   ArF represents a fluoroaryl group or a fluoroheteroaryl group,
   each of R^{a} and R^{b} independently represents a hydrogen atom (H), an alkyl group, a benzyl group, an aryl group or a heteroaryl group, and
   A represents an anion.)
[2] Another aspect of the present invention relates to the organic electronic material described above, wherein the anion is represented by one of general formulas (1b) to (5b) shown below. 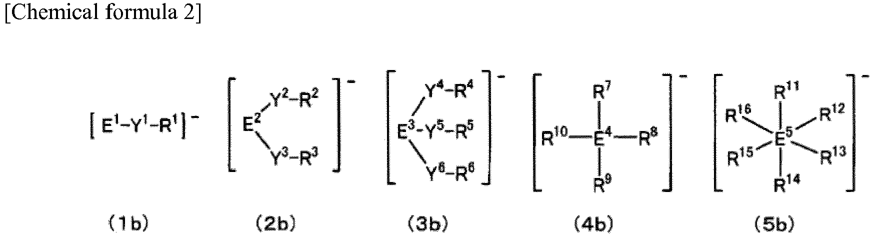 (In general formulas (1b) to (5b):
   each of Y¹ to Y⁶ independently represents a single bond or a divalent linking group,
   each of R¹ to R¹⁶ independently represents an electron-withdrawing monovalent group, wherein R² and R³, at least two groups selected from among R⁴ to R⁶, at least two groups selected from among R⁷ to R¹⁰, and at least two groups selected from among R¹¹ to R¹⁶, may each be bonded together to form a ring, and
   E¹ represents an oxygen atom, E² represents a nitrogen atom, E³ represents a carbon atom, E⁴ represents a boron atom or a gallium atom, and E⁵ represents a phosphorus atom or an antimony atom.)
[3] Yet another aspect of the present invention relates to the organic electronic material described above, wherein at least one of R^{a} and R^{b} is an alkyl group, a benzyl group, an aryl group or a heteroaryl group.
[4] Yet another aspect of the present invention relates to the organic electronic material described above, wherein at least one of R^{a} and R^{b} is an alkyl group or a benzyl group.
[5] Yet another aspect of the present invention relates to the organic electronic material described above, wherein ArF is a fluoroaryl group.
[6] Yet another aspect of the present invention relates to the organic electronic material described above, wherein the charge transport unit is an aromatic amine, a carbazole or a thiophene.
[7] Yet another aspect of the present invention relates to the organic electronic material described above, wherein the compound having a charge transport unit is a polymer or an oligomer.
[8] Yet another aspect of the present invention relates to the organic electronic material described above, wherein the compound having a charge transport unit has at least one polymerizable functional group.
[9] Yet another aspect of the present invention relates to the organic electronic material described above, wherein the polymerizable functional group is at least one group selected from the group consisting of an oxetanyl group, an epoxy group and a vinyl ether group.
[10] A separate aspect of the present invention relates to an ink composition containing the organic electronic material described above and a solvent.
[11] Another separate aspect of the present invention relates to an organic electronic element containing an organic layer formed using the organic electronic material described above or the ink composition described above.
[12] Another aspect of the present invention relates to the organic electronic element described above, wherein the organic electronic element has multilayered organic layers produced by forming a separate organic layer on top of the organic layer described above.
[13] Yet another aspect of the present invention relates to the organic electronic element described above, wherein at least one of the organic layer described above and the separate organic layer described above is at least one layer selected from the group consisting of a hole injection layer, a hole transport layer and a light-emitting layer.
[14] Yet another aspect of the present invention relates to the organic electronic element described above, further containing a substrate, wherein
   the substrate is a resin film.
[15] Yet another aspect of the present invention relates to the organic electronic element described above, wherein the organic electronic element is an organic electroluminescent element.
[16] Another separate aspect of the present invention relates to a method for producing an organic electronic element that includes a step of forming an organic layer by a coating method using the organic electronic material described above or the ink composition described above.
[17] Another aspect of the present invention relates to the method for producing an organic electronic element described above, further including a step of polymerizing and insolubilizing the organic layer formed by a coating method.
[18] Yet another aspect of the present invention relates to the method for producing an organic electronic element described above, further including a step of performing multilayering by forming a separate organic layer on top of the insolubilized organic layer.

### EFFECTS OF THE INVENTION

Embodiments of the present invention are able to provide an organic electronic material capable of forming an organic electronic element having a low drive voltage and excellent emission efficiency and lifespan characteristics.

### BRIEF DESCRIPTION OF THE DRAWINGS

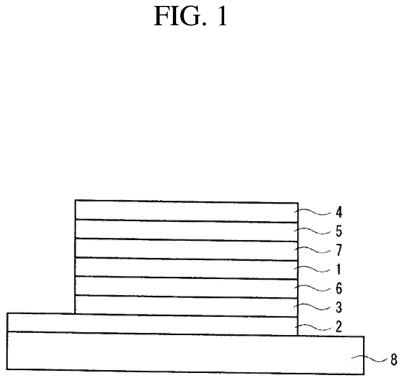

FIG. 1 is a cross-sectional schematic view illustrating one example of an organic EL element of one embodiment of the present invention.

### EMBODIMENTS FOR CARRYING OUT THE INVENTION

### <Organic Electronic Material>

An organic electronic material of one embodiment of the present invention contains an ionic compound represented by general formula (1) shown below, and a compound having a charge transport unit (hereafter also referred to as a charge transport compound). 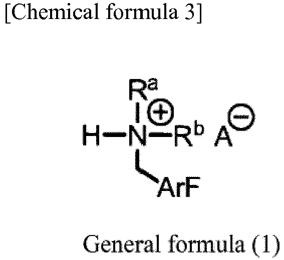 In general formula (1):
ArF represents a fluoroaryl group or a fluoroheteroaryl group,
each of R^{a} and R^{b} independently represents a hydrogen atom (H), an alkyl group, a benzyl group, an aryl group or a heteroaryl group, and
A represents an anion.

In the present embodiment, the ionic compound represented by the above general formula (1) is characterized in that of the four groups bonded to the N (nitrogen atom) that constitutes the cation site, at least one group is a hydrogen atom, and at least one group is a -CH₂-Ar-F group, namely a group composed of a methylene group, an arylene or heteroarylene group, and a fluoro group.

By using the ionic compound represented by general formula (1), the drive voltage of an organic electronic element formed using the organic electronic material of the present embodiment can be reduced, and the emission efficiency and lifespan characteristics can be enhanced.

Although the reasons that these types of effects are achieved is not entirely clear, they are thought to include the following. In the organic electronic material of the present embodiment, it is thought that by including a charge transport compound and the ionic compound of general formula (1) having a specific cation structure, doping of the charge transport compound occurs rapidly. As a result, it is thought that the hole density increases, yielding an improvement in the charge transport properties.

Accordingly, a layer formed using the organic electronic material of the present embodiment (hereafter also referred to as an "organic layer") exhibits excellent charge transport properties. As a result, it is thought that by using an organic electronic element having such a layer, a reduction in the power consumption of the element, and an improvement in the emission efficiency and lifespan characteristics of the element can be achieved. It should be noted that this mechanism is merely a theory, and in no way limits the present invention.

Further, in one embodiment, as described below, using the ionic compound represented by general formula (1) offers the advantage that an improvement in the charge transport properties can be easily obtained, even in those cases where a charge transport compound having a deep highest occupied molecular orbital (HOMO) is used.

Furthermore, in one embodiment, by using a combination of a charge transport compound having a polymerizable functional group and the ionic compound represented by general formula (1), the curing properties of the organic electronic material can be improved. It is thought that this is due to the ionic compound represented by general formula (1) functioning as a polymerization initiator. As a result, multilayering of organic layers in organic electronic elements can be performed more easily. Accordingly, combining the ionic compound represented by general formula (1) and a charge transport compound having a polymerizable functional group also offers the advantage that the organic electronic material can be used favorably in the production of stacked elements using coating methods.

Examples of the compound represented by general formula (1) are described below in further detail.

In general formula (1), more specifically, ArF represents a fluoroaryl group or a fluoroheteroaryl group, wherein each of these groups may have a substituent. Each of R^{a} and R^{b} independently represents a hydrogen atom (H), or an alkyl group, benzyl group, aryl group or heteroaryl group, wherein each of these groups may have a substituent. A represents an anion.

The substituents on the fluoroaryl group and fluoroheteroaryl group are preferably each independently an alkyl group or an alkoxy group. The substituents on the alkyl group and benzyl group are preferably each independently a halogen, whereas the substituents on the aryl group and heteroaryl group are preferably each independently a halogen, an alkyl group or an alkoxy group.

From the viewpoint of improving the solubility in solvents when the organic electronic material of the present embodiment is converted to an ink composition, at least one of R^{a} and R^{b} in general formula (1) is preferably an alkyl group, benzyl group, aryl group or heteroaryl group. Further, it is more preferable that at least one of R^{a} and R^{b} is an alkyl group or a benzyl group (in other words, those cases where R^{a} and R^{b} are both aryl groups or heteroaryl groups are excluded). It is even more preferable that R^{a} and R^{b} are both either an alkyl group or a benzyl group. These groups may be unsubstituted or may have a substituent.

The groups represented by ArF, R^{a} and R^{b} in general formula (1) are described below in further detail.

The fluoroaryl group represented by ArF is a group in which at least one hydrogen atom of an aryl group has been substituted with a fluorine atom. The fluoroheteroaryl group represented by ArF is a group in which at least one hydrogen atom of a heteroaryl group has been substituted with a fluorine atom.

The number n of fluoro groups in the group represented by ArF (the number of substituted fluorine atoms) may be 1 or greater, and all of the positions that can be substituted within the aryl group or heteroaryl group may be substituted. From the viewpoint of the charge transport properties, the number n of fluoro groups is preferably at least 1 but not more than 10, and is more preferably not more than 7, and even more preferably 5 or fewer.

The aryl group in ArF is an atom grouping in which one hydrogen atom has been removed from an aromatic hydrocarbon. The aromatic hydrocarbon includes not only monocyclic aromatic hydrocarbons, but also condensed ring hydrocarbons, and hydrocarbons in which two or more independent benzene rings or condensed ring systems are bonded together, either directly or via a divalent group such as a vinylene group. A benzene ring or an aromatic hydrocarbon composed of 2 to 4 condensed aromatic rings is preferred. Further, the aryl group may have a substituent, and examples of the substituent include halogens other than fluorine (for example, chlorine, bromine, and iodine and the like), alkoxy groups and alkyl groups. The number of carbon atoms in the aryl group (including carbon atoms in any substituents) is typically from 6 to about 60, and is preferably from 6 to 30, more preferably from 6 to 20, and particularly preferably from 6 to 15.

Specific examples include a phenyl group, C₁ to C₁₂ alkoxyphenyl groups (wherein the C₁ to C₁₂ means that the number of carbon atoms in the substituent is from 1 to 12, with this numbering convention also used below), C₁ to C₁₂ alkylphenyl groups, and a 1-naphthyl group, 2-naphthyl group, 1-anthracenyl group, 2-anthracenyl group, 9-anthracenyl group, phenanthrenyl group, pyrenyl group, perylenyl group and pentafluorophenyl group. Of these, a phenyl group, C₁ to C₁₂ alkoxyphenyl group or C₁ to C₁₂ alkylphenyl group is preferred, and a phenyl group, C₁ to C₅ alkoxyphenyl group or C₁ to C₅ alkylphenyl group is more preferred. A phenyl group is particularly desirable.

Specific examples of the C₁ to C₁₂ alkoxy group include methoxy, ethoxy, propyloxy, i-propyloxy, butoxy, i-butoxy, t-butoxy, pentyloxy, hexyloxy, cyclohexyloxy, heptyloxy, octyloxy, 2-ethylhexyloxy, nonyloxy, decyloxy, 3,7-dimethyloctyloxy and lauryloxy groups. Further, specific examples of the C₁ to C₁₂ alkyl group include methyl, ethyl, propyl, i-propyl, butyl, i-butyl, t-butyl, pentyl, hexyl, cyclohexyl, heptyl, octyl, 2-ethylhexyl, nonyl, decyl, 3,7-dimethyloctyl and lauryl groups.

The heteroaryl group in ArF is an atom grouping that remains when one hydrogen atom is removed from an aromatic heterocyclic compound. The heteroaryl group may be unsubstituted or may have a substituent. Examples of the substituent include halogens other than fluorine (for example, chlorine, bromine, and iodine and the like), and the alkyl groups and alkoxy groups described above in relation to the aryl group. An alkyl group is preferred. The number of carbon atoms in an unsubstituted monovalent aromatic heterocyclic group is typically from 4 to about 60, and is preferably from 4 to 20. Among the various possibilities, groups having a 4- to 6-membered hetero ring are particularly preferred. Examples of the hetero atom include S (sulfur), O (oxygen) and N (nitrogen).

Specific examples of monovalent heterocyclic groups include a thienyl group, C₁ to C₁₂ alkylthienyl groups, pyrrolyl group, furyl group, pyridyl group and C₁ to C₁₂ alkylpyridyl groups. Of these, a thienyl group, C₁ to C₁₂ alkylthienyl group, pyridyl group or C₁ to C₁₂ alkylpyridyl group is preferred, and a C₁ to C₅ alkylthienyl group, pyridyl group or C₁ to C₅ alkylpyridyl group is particularly preferred.

The group represented by ArF is preferably a fluoroaryl group, and is more preferably a fluorophenyl group.

The alkyl group represented by R^{a} or R^{b} may be linear, branched or cyclic. The alkyl group may also have a substituent. Examples of the substituent include halogens (for example, fluorine, chlorine, bromine, and iodine and the like), C₁ to C₁₂ alkoxy groups, and an aldehyde group, carboxyl group, amino group, sulfo group, hydroxyl group and nitro group, but halogen atoms are preferred.

The number of carbon atoms in the alkyl group (including carbon atoms in any substituents) is typically from 1 to about 20, and is preferably from 1 to 15. Further, at least one of R^{a} and R^{b} is preferably an alkyl group of 1 to 6 carbon atoms. Specific examples of the alkyl group include a methyl group, ethyl group, propyl group, i-propyl group, butyl group, i-butyl group, t-butyl group, pentyl group, hexyl group, cyclohexyl group, heptyl group, octyl group, 2-ethylhexyl group, nonyl group, 3,7-dimethyloctyl group, lauryl group, trifluoromethyl group, pentafluoroethyl group, perfluorobutyl group, perfluorohexyl group and perfluorooctyl group.

The benzyl group represented by R^{a} or R^{b} may have a substituent. Examples of the substituent include halogens (such as fluorine, chlorine, iodine, and bromine and the like), C₁ to C₁₂ alkoxy groups, and an aldehyde group, carboxyl group, amino group, sulfo group, hydroxyl group and nitro group, but halogen atoms are preferred, and a fluorine is particularly preferred.

Examples of the aryl groups and heteroaryl groups represented by R^{a} or R^{b} include the same groups as those listed above as the aryl group and heteroaryl group in ArF. The aryl group or heteroaryl group represented by R^{a} or R^{b} may be unsubstituted, or may have a substituent. Examples of the substituent include the groups listed above as substituents for the aryl group and heteroaryl group in ArF, and fluorine.

Examples of the combination of R^{a} and R^{b} include, but are not limited to, a combination of an alkyl group of 1 to 6 carbon atoms and a benzyl group or fluorobenzyl group, a combination of an alkyl group of 1 to 6 carbon atoms and another alkyl group of 1 to 6 carbon atoms, and a combination of an alkyl group of 1 to 6 carbon atoms and an alkyl group of 7 to 20 carbon atoms.

In general formula (1), the anion represented by A is not particularly limited, and may be any anion known within the technical field. In one embodiment, from the viewpoint of producing an organic electronic element, and particularly an organic EL element, that is capable of achieving a reduction in drive voltage and stable long-term operation, an anion represented by one of general formulas (1b) to (5b) shown below is preferred. 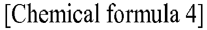 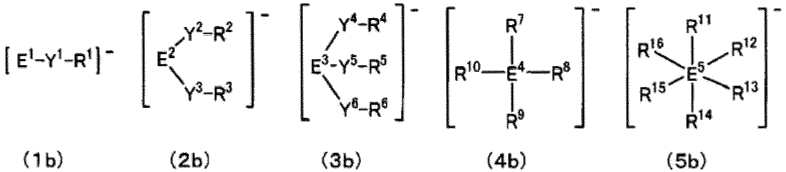 In general formulas (1b) to (5b):
each of Y¹ to Y⁶ independently represents a single bond or a divalent linking group,
each of R¹ to R¹⁶ independently represents an electron-withdrawing monovalent group. A substituent and/or a hetero atom may also be included within the structure of these groups. Further, the groups R² and R³, R⁴ to R⁶, R⁷ to R¹⁰, or R¹¹ to R¹⁶, may be bonded together to form a ring, or may form a polymer structure.
E¹ represents an oxygen atom, E² represents a nitrogen atom, E³ represents a carbon atom, E⁴ represents a boron atom or a gallium atom, and E⁵ represents a phosphorus atom or an antimony atom.

It is preferable that in one embodiment of the anion represented by A, in general formulas (1b) to (5b), each of Y¹ to Y⁶ independently represents a single bond or a divalent linking group, each of R¹ to R¹⁶ independently represents an electron-withdrawing monovalent group, wherein R² and R³, at least two groups selected from among R⁴ to R⁶, at least two groups selected from among R⁷ to R¹⁰, and at least two groups selected from among R¹¹ to R¹⁶, may each be bonded together to form a ring, and E¹ represents an oxygen atom, E² represents a nitrogen atom, E³ represents a carbon atom, E⁴ represents a boron atom or a gallium atom, and E⁵ represents a phosphorus atom or an antimony atom. Further, in another embodiment of the anion represented by A, two or more structures each represented by one of general formulas (1b) to (5b) may be linked together via one of R¹ to R¹⁶ to form a polymer structure.

Examples of the electron-withdrawing monovalent group (R¹ to R¹⁶ in the above formulas) include halogen atoms such as a fluorine atom, chlorine atom and bromine atom; a cyano group; a thiocyano group; a nitro group; alkylsulfonyl groups such as a mesyl group; arylsulfonyl groups such as a tosyl group; acyl groups typically having at least 1 but not more than 12 carbon atoms, and preferably not more than 6 carbon atoms, such as a formyl group, acetyl group and benzoyl group; alkoxycarbonyl groups typically having at least 2 but not more than 10 carbon atoms, and preferably not more than 7 carbon atoms, such as a methoxycarbonyl group and an ethoxycarbonyl group; aryloxycarbonyl groups having an aromatic hydrocarbon group or an aromatic heterocyclic group typically having at least 3 but not more than 25 carbon atoms, and preferably at least 4 but not more than 15 carbon atoms, such as a phenoxycarbonyl group and a pyridyloxycarbonyl group; acyloxy groups typically having at least 2 but not more than 20 carbon atoms, such as an acetoxy group; alkyloxysulfonyl groups; aryloxysulfonyl groups; haloalkyl groups, haloalkenyl groups and haloalkynyl groups in which an alkyl group, alkenyl group or alkynyl group has been substituted with a halogen atom such as a fluorine atom or chlorine atom, such as a trifluoromethyl group and a pentafluoroethyl group; and haloaryl groups typically having at least 6 but not more than 20 carbon atoms such as a pentafluorophenyl group. In the above haloalkyl groups, haloalkenyl groups and haloalkynyl groups, the alkyl group, alkenyl group or alkynyl group may be a linear, branched or cyclic group typically having at least 1 but not more than 20 carbon atoms, preferably at least 1 but not more than 10 carbon atoms, and more preferably not more than 6 carbon atoms, and may also include a hetero atom (such as N, O or S) within the structure.

Among these groups, from the viewpoint of enabling efficient delocalization of the negative charge, groups among the monovalent groups listed above in which some or all of the hydrogen atoms of a group having hydrogen atoms have each been substituted with a halogen atom such as a fluorine atom are particularly preferred. Specific examples of such groups include perfluoroalkyl groups, perfluoroalkylsulfonyl groups, perfluoroaryl groups, perfluoroalkyloxysulfonyl groups, perfluoroarylsulfonyl groups, perfluoroaryloxysulfonyl groups, perfluoroacyl groups, perfluoroalkoxycarbonyl groups, perfluoroacyloxy groups, perfluoroaryloxycarbonyl groups, perfluoroalkenyl groups and perfluoroalkynyl groups. These groups preferably have 1 to 20 carbon atoms, may also include a hetero atom (such as N, O or S), and may be linear, branched or cyclic.

Specific examples include, but are not limited to, groups represented by a structural formula series (1) shown below. Further, among these structural formulas, linear or branched perfluoroalkyl groups of 1 to 8 carbon atoms, cyclic perfluoroalkyl groups of 3 to 6 carbon atoms, and perfluoroaryl groups of 6 to 18 carbon atoms are preferred.

Structural Formula Series (1)

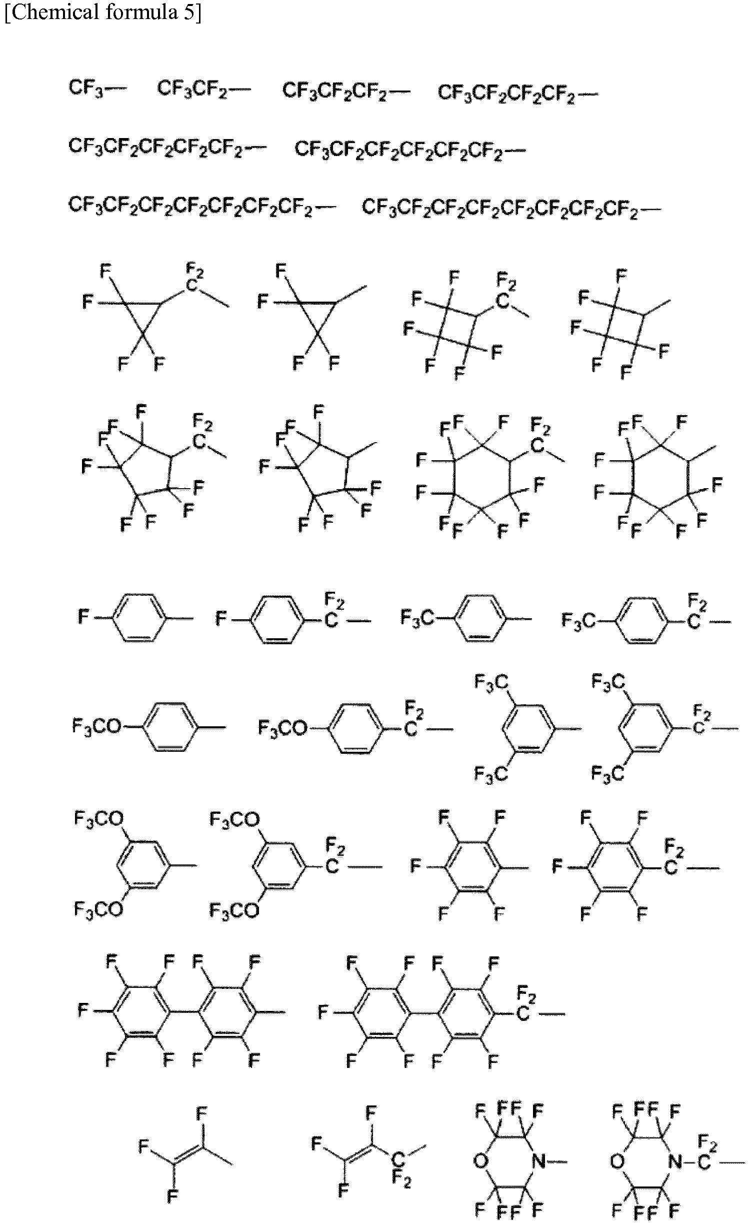

Further, in those cases where Y¹ to Y⁶ in the above general formulas represent divalent linking groups, specific examples of the linking group include any one of general formulas (1c) to (11c) shown below.

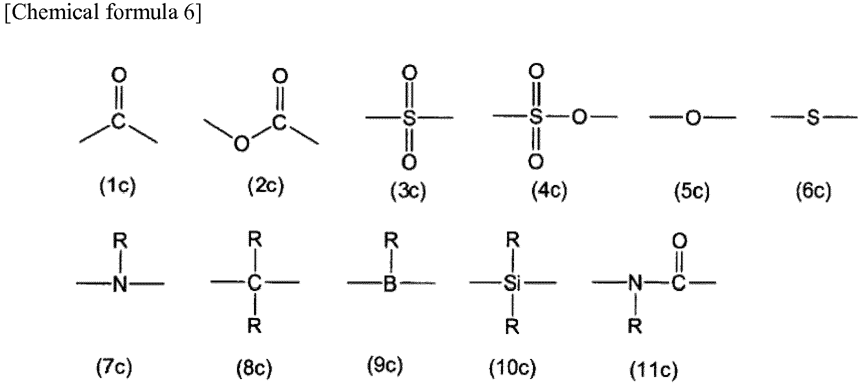

In the above formulas, R represents an arbitrary organic group.

From the viewpoints of enhancing the electron-accepting properties and improving the solubility in solvents, each R in general formulas (7c) to (11c) preferably independently represents an alkyl group, alkenyl group, alkynyl group, aromatic hydrocarbon group or aromatic heterocyclic group. These groups may also include a substituent and/or a hetero atom within the structure. Examples of particularly preferred groups include organic groups having an electron-withdrawing substituent selected from among the monovalent groups listed above in relation to the general formulas (1b) to (5b), and specific examples include groups of the above structural formula series (1).

Further, the anion represented by A in general formula (1) is preferably an anion in which the negative charge resides mainly on an oxygen atom, nitrogen atom, carbon atom, boron atom or gallium atom. Although there are no particular limitations, the anion is more preferably an anion in which the negative charge resides mainly on an oxygen atom, nitrogen atom, carbon atom or boron atom, and is most preferably an anion represented by one of general formulas (12c), (13c), (14c) and (15c) shown below. 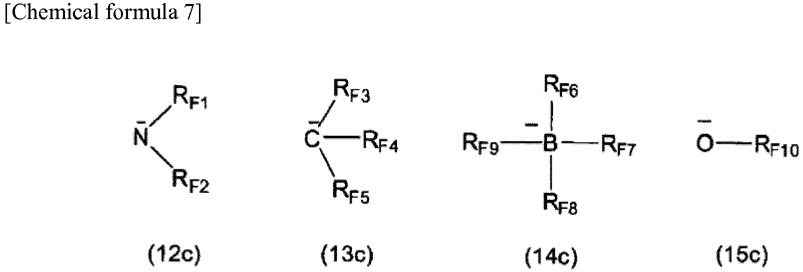 (In the formulas, each of R_{F1} to R_{F10} independently represents an electron-withdrawing monovalent group, and a substituent and/or hetero atom may also be included within any of these groups. Further, groups among R_{F1} to R_{F9} may be bonded together to form either a ring or a polymer structure.

Specifically, in one embodiment of the anion represented by A, R_{F1} and R_{F2}, at least two groups selected from among R_{F3} to R_{F5}, or at least two groups selected from among R_{F6} to R_{F9}, may be bonded together to form a ring. Further, in another embodiment of the anion represented by A, two or more structures each represented by one of general formulas (12c) to (15c) may be linked together via one of R_{F1} to R_{F10} to form a polymer structure.

There are no particular limitations on examples of R_{F1} to R_{F10}, and specific examples include the groups shown in the structural formula series (1).

Although there are no particular limitations on the amount of the ionic compound represented by general formula (1), the amount is preferably at least 0.01% by mass but not more than 20% by mass, within the total mass of the organic electronic material. An amount of at least 0.01% by mass is preferred from the viewpoints of the charge transport properties and the curability, whereas an amount of not more than 20% by mass is preferred from the viewpoint of film formability. The amount of the ionic compound within the total mass of the organic electronic material is more preferably at least 0.1% by mass, and even more preferably 0.5% by mass or greater, and is more preferably not more than 20% by mass, and even more preferably 10% by mass or less.

### [Charge Transport Compound]

The "charge transport compound" in the present embodiment is described below in further detail. In the present embodiment, the term "charge transport compound" refers to a compound having a charge transport unit. In the present embodiment, a "charge transport unit" is an atom grouping that has the ability to transport a positive hole or an electron, and details of that atom grouping are described below.

There are no particular limitations on the above charge transport unit, provided it has the ability to transport a positive hole or an electron, but amines having an aromatic ring, carbazoles and thiophenes are preferred. Specific examples include the structures disclosed in WO 2011/132702. Amine structures (1) to (14) shown below are particularly preferred. Examples of E, Ar and X in the following amine structures (1) to (14) include those groups disclosed in the above publication. Specifically, E, Ar and X are as follows.

Each E independently represents -R¹, -OR², -SR³, -OCOR⁴, -COOR⁵, or -SiR⁶R⁷R⁸ or the like. Here, each of R¹ to R⁸ represents a hydrogen atom, a linear, cyclic or branched alkyl group of 1 to 22 carbon atoms, or an aryl group or heteroaryl group of 2 to 30 carbon atoms.

Each Ar independently represents an arylene group or heteroarylene group of 2 to 30 carbon atoms. The arylene group or heteroarylene group may also have a substituent.

Each X independently represents a divalent linking group, and although there are no particular limitations, preferred groups include groups in which one hydrogen atom has been removed from those groups having at least one hydrogen atom described above in relation to E.

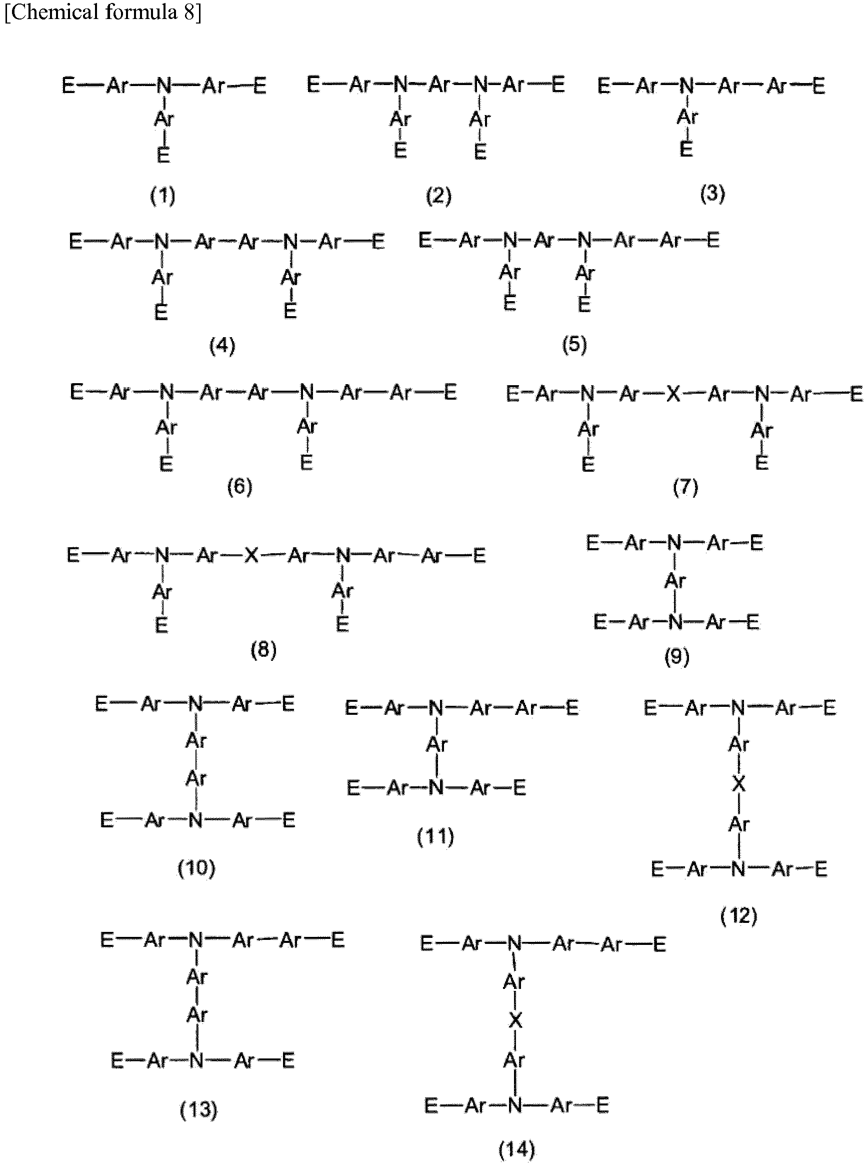

Further, the charge transport compound in the present embodiment may be a low-molecular weight compound, or a high-molecular weight compound such as a polymer or oligomer. A high-molecular weight compound such as a polymer or oligomer is preferred from the viewpoint of the solubility in organic solvents, whereas a low-molecular weight compound is preferred from the viewpoint of offering simple purification by sublimation or recrystallization or the like.

In those cases where the charge transport compound in the present embodiment is a polymer or oligomer, from the viewpoint of lowering the temperature required to ensure that the polymerization reaction proceeds satisfactorily, a polymer or oligomer having a structure that is branched in at least three directions is preferred. Further, this branched structure enables the glass transition temperature of the polymer or oligomer to be increased, contributing to an improvement in the heat resistance of the polymer or oligomer.

This branched structure means that among the various chains within a single molecule of the polymer or oligomer, if the chain that has the highest degree of polymerization is deemed the main chain, then a side chain having a degree of polymerization that is either the same as, or smaller than, that of the main chain is linked to the main chain. In this embodiment, the "degree of polymerization" represents the number of monomer units used in synthesizing the polymer or oligomer that are contained within one molecule of the polymer or oligomer. Further, in this embodiment, a "side chain" means a chain that is different from the main chain of the polymer or oligomer and has at least one polymer unit, whereas other moieties outside of this definition are deemed substituents.

There are no particular limitations on the method used for forming the branched structure, and either the polymer or oligomer may be formed using a monomer that has three or more polymerizable sites within a single molecule, or a linear polymer or oligomer may be formed first, and the branched structure then formed by polymerizing the linear polymer or oligomer chains.

Specifically, a structural unit B described below is preferably included as a unit capable of functioning as a starting point for forming a branched structure within the polymer or oligomer.

Furthermore, in order to enable adjustment of the degree of solubility, the heat resistance and the electrical properties, the polymer or oligomer in the present embodiment may be a copolymer which, in addition to the repeating units represented by general formulas (1a) to (84a) disclosed in the above publication WO 2011/132702, also has a structure represented by a structural unit L described below as the aforementioned arylene group or heteroarylene group, which functions as a copolymer repeating unit. In such cases, the copolymer may be a random, block or graft copolymer, or may be a copolymer having an intermediate type structure, such as a random copolymer having block-like properties. Further, the polymer or oligomer used in the present embodiment may have a branch within the main chain, and have three or more terminals.

Further, the charge transport compound in the present embodiment is not limited to the compounds described above, and both commercially available compounds and compounds synthesized using known methods may be used without any particular limitations.

Specific examples of polymers and oligomers that may be used in the organic electronic material of the present embodiment are described below. In the following description, the polymer or oligomer is sometimes referred to as a "charge transport polymer".

The charge transport polymer may be linear, or may have a branched structure. The charge transport polymer preferably has at least a divalent structural unit L that has charge transport properties, and a monovalent structural unit T that constitutes the terminal portions, and may also have a trivalent or higher-valent structural unit B that constitutes a branched portion. The charge transport polymer may contain only one type of each of these structural units, or may contain a plurality of types of each structural unit. In the charge transport polymer, the various structural units are bonded together at "monovalent" to "trivalent or higher-valent" bonding sites.

### (Structure)

Examples of the partial structures contained in the charge transport polymer include those shown below. However, the charge transport polymer is not limited to polymers having the following partial structures. In the partial structures, "L" represents a structural unit L, "T" represents a structural unit T, and "B" represents a structural unit B. The symbol "*" denotes a bonding site to another structural unit. In the following partial structures, the plurality of L structural units may be units having the same structure or units having mutually different structures. This also applies for the T and B units.

### Linear Charge Transport Polymer

[Chemical formula 9] T-L-L-L-L-L-∗

### Charge Transport Polymers having Branched Structures

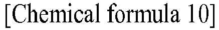

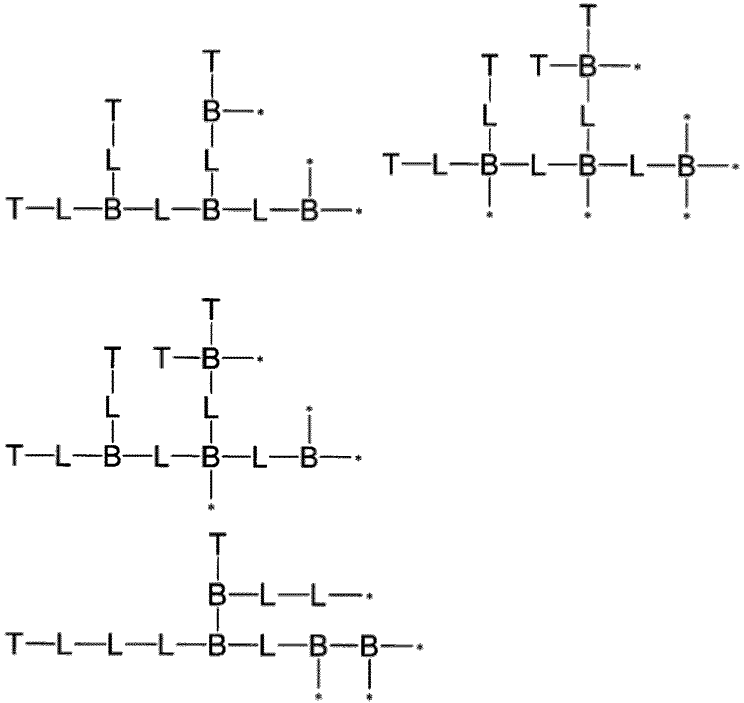

### (Structural Unit L)

The structural unit L is preferably a divalent structural unit having charge transport properties. There are no particular limitations on the structural unit L, provided the structural unit includes an atom grouping having the ability to transport a charge. For example, the structural unit L may be selected from among substituted or unsubstituted structures, including aromatic amine structures, carbazole structures, thiophene structures, fluorene structures, benzene structures, biphenylene structures, terphenylene structures, naphthalene structures, anthracene structures, tetracene structures, phenanthrene structures, dihydrophenanthrene structures, pyridine structures, pyrazine structures, quinoline structures, isoquinoline structures, quinoxaline structures, acridine structures, diazaphenanthrene structures, furan structures, pyrrole structures, oxazole structures, oxadiazole structures, thiazole structures, thiadiazole structures, triazole structures, benzothiophene structures, benzoxazole structures, benzoxadiazole structures, benzothiazole structures, benzothiadiazole structures, benzotriazole structures, and structures containing one type, or two or more types, of the above structures. The aromatic amine structures are preferably triarylamine structures, and more preferably triphenylamine structures.

In one embodiment, from the viewpoint of obtaining superior hole transport properties, the structural unit L is preferably selected from among substituted or unsubstituted structures including aromatic amine structures, carbazole structures, thiophene structures, fluorene structures, benzene structures, pyrrole structures, and structures containing one type, or two or more types, of these structures, and is more preferably selected from among substituted or unsubstituted structures including aromatic amine structures, carbazole structures, and structures containing one type, or two or more types, of these structures.

Specific examples of the structural unit L are shown below. However, the structural unit L is not limited to the following structures.

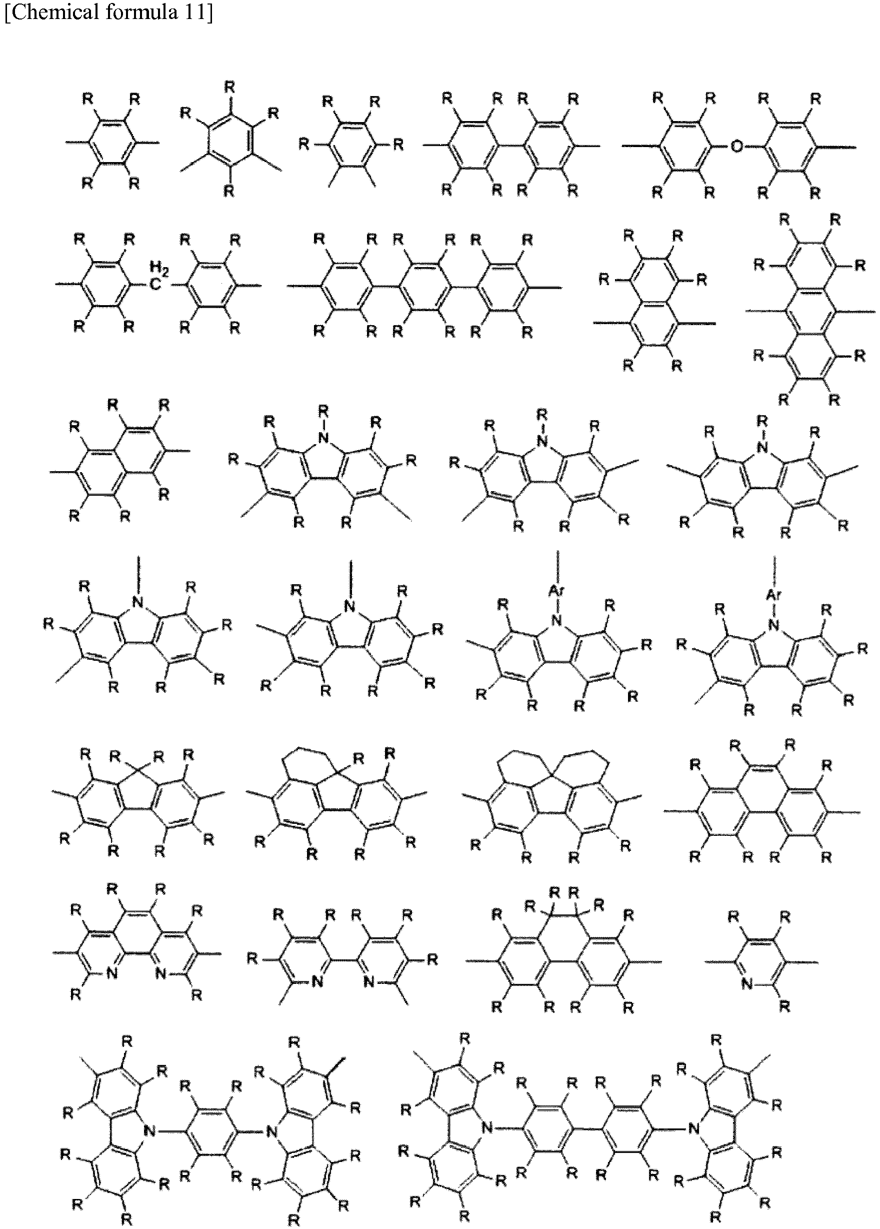

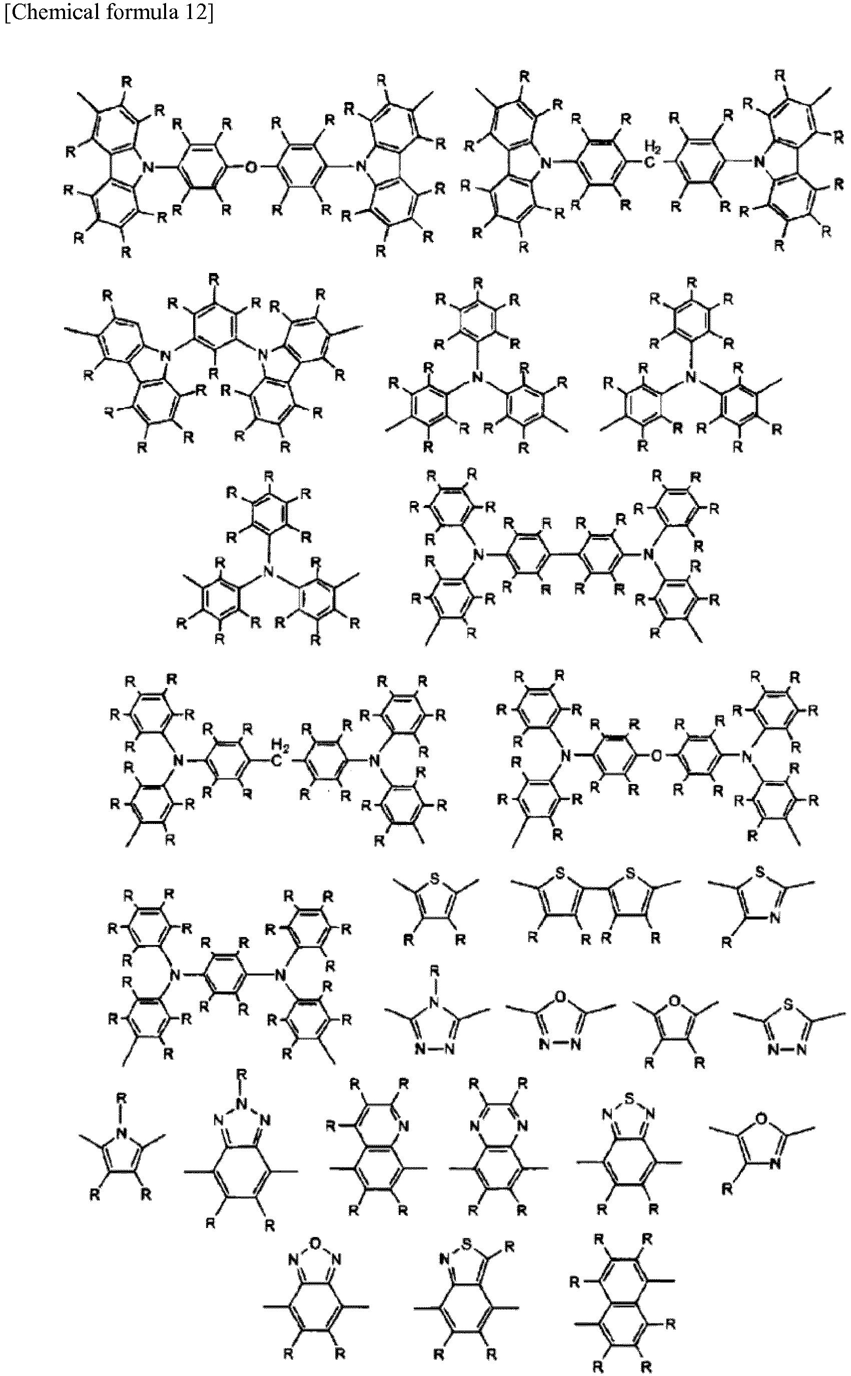

Each R in the above structural units independently represents a hydrogen atom or a substituent. It is preferable that each R is independently selected from a group consisting of -R¹, -OR², -SR³, - OCOR⁴, -COOR⁵, -SiR⁶R⁷R⁸, halogen atoms, and groups containing a polymerizable functional group described below. Each of R¹ to R⁸ independently represents a hydrogen atom, a linear, cyclic or branched alkyl group of 1 to 22 carbon atoms, or an aryl group or heteroaryl group of 2 to 30 carbon atoms. An aryl group is an atom grouping in which one hydrogen atom has been removed from an aromatic hydrocarbon. A heteroaryl group is an atom grouping in which one hydrogen atom has been removed from an aromatic heterocycle. The alkyl group may be further substituted with an aryl group or heteroaryl group of 2 to 20 carbon atoms, and the aryl group or heteroaryl group may be further substituted with a linear, cyclic or branched alkyl group of 1 to 22 carbon atoms. R is preferably a hydrogen atom, an alkyl group, an aryl group, or an alkyl-substituted aryl group. Ar represents an arylene group or heteroarylene group of 2 to 30 carbon atoms. An arylene group is an atom grouping in which two hydrogen atoms have been removed from an aromatic hydrocarbon. A heteroarylene group is an atom grouping in which two hydrogen atoms have been removed from an aromatic heterocycle. Ar is preferably an arylene group, and is more preferably a phenylene group.

Examples of the aromatic hydrocarbon include monocyclic hydrocarbons, condensed ring hydrocarbons, and polycyclic hydrocarbons in which two or more hydrocarbons selected from among monocyclic hydrocarbons and condensed ring hydrocarbons are bonded together via single bonds. Examples of the aromatic heterocycle include monocyclic heterocycles, condensed ring heterocycles, and polycyclic heterocycles in which two or more heterocycles selected from among monocyclic heterocycles and condensed ring heterocycles are bonded together via single bonds.

### (Structural Unit B)

The structural unit B is a trivalent or higher-valent structural unit that constitutes a branched portion in those cases where the charge transport polymer has a branched structure. From the viewpoint of improving the durability of the organic electronic element, the structural unit B is preferably not higher than hexavalent, and is more preferably either trivalent or tetravalent. The structural unit B is preferably a unit that has charge transport properties. For example, from the viewpoint of improving the durability of the organic electronic element, the structural unit B is preferably selected from among substituted or unsubstituted structures including aromatic amine structures, carbazole structures, condensed polycyclic aromatic hydrocarbon structures, and structures containing one type, or two or more types, of these structures.

Specific examples of preferred structural units B include units containing one of the structures represented by general formulas (1) to (10) shown below. However, the structural unit B is not limited to the following structures.

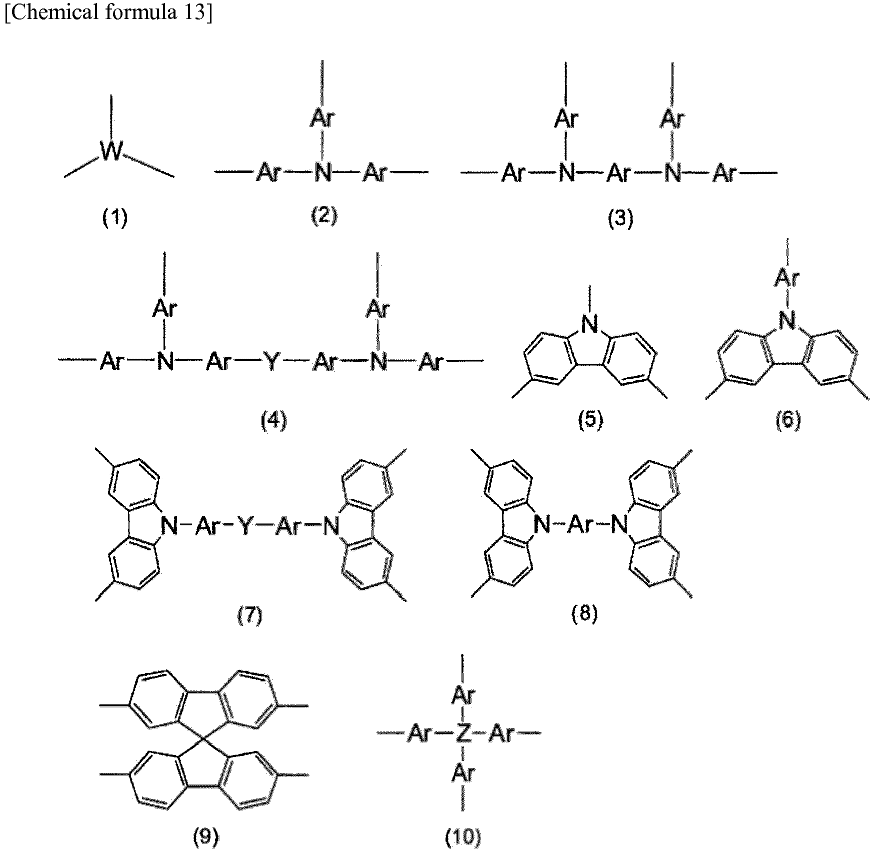

In the above formulas, each Ar independently represents a divalent linking group, and for example, may represent an arylene group or heteroarylene group of 2 to 30 carbon atoms. Ar is preferably an arylene group, and more preferably a phenylene group.

An arylene group is an atom grouping in which two hydrogen atoms have been removed from an aromatic hydrocarbon, and may have a substituent. Examples include a phenylene group, and biphenyl-diyl, terphenyl-diyl, naphthalene-diyl, anthracene-diyl, tetracene-diyl, fluorene-diyl and phenanthrene-diyl groups.

A heteroarylene group is an atom grouping in which two hydrogen atoms have been removed from an aromatic compound having a hetero atom, and may have a substituent. Examples include pyridine-diyl, pyrazine-diyl, quinoline-diyl, isoquinoline-diyl, acridine-diyl, phenanthroline-diyl, furan-diyl, pyrrole-diyl, thiophene-diyl, oxazole-diyl, oxadiazole-diyl, thiadiazole-diyl, triazole-diyl, benzoxazole-diyl, benzoxadiazole-diyl, benzothiadiazole-diyl, benzotriazole-diyl and benzothiophenediyl groups.

W represents a trivalent linking group, and for example, represents an atom grouping in which an additional one hydrogen atom has been removed from an aforementioned arylene group or heteroarylene group, which may have a substituent. Examples include an arenetriyl group or heteroarenetriyl group of 2 to 30 carbon atoms. An arenetriyl group is an atom grouping in which three hydrogen atoms have been removed from an aromatic hydrocarbon. A heteroarenetriyl is an atom grouping in which three hydrogen atoms have been removed from an aromatic heterocycle.

Each Y independently represents a divalent linking group. Examples include divalent groups in which an additional hydrogen atom has been removed from any of the R groups having one or more hydrogen atoms (but excluding groups containing a polymerizable functional group) described in relation to the structural unit L. Z represents a carbon atom, a silicon atom or a phosphorus atom. In the above structural units, the benzene rings and Ar groups may have substituents, and examples of the substituents include the R groups in the structural unit L.

The Y groups in the above general formulas (4) and (7) are preferably divalent linking groups represented by one of the following formulas. 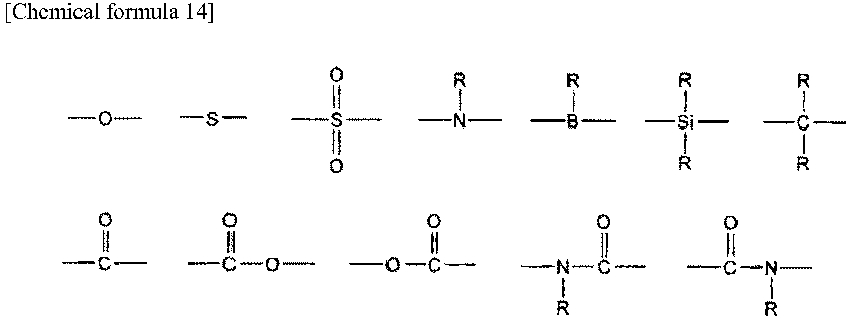 In the formulas, each R independently represents a hydrogen atom, a linear, cyclic or branched alkyl group of 1 to 22 carbon atoms, or an aryl group or heteroaryl group of 2 to 30 carbon atoms. Here, an aryl group is an atom grouping in which one hydrogen atom has been removed from an aromatic hydrocarbon, and may have a substituent, whereas a heteroaryl group is an atom grouping in which one hydrogen atom has been removed from an aromatic compound having a hetero atom, and may have a substituent.

### (Structural Unit T)

The structural unit T is a monovalent structural unit that constitutes a terminal portion of the charge transport polymer. There are no particular limitations on the structural unit T, which may, for example, be selected from among substituted or unsubstituted structures including aromatic hydrocarbon structures, aromatic heterocyclic structures, and structures containing one type, or two or more types, of these structures. The structural unit T may have a similar structure to the structural unit L. In one embodiment, from the viewpoint of imparting durability without impairing the charge transport properties, the structural unit T is preferably a substituted or unsubstituted aromatic hydrocarbon structure, and is more preferably a substituted or unsubstituted benzene structure. Further, in another embodiment, in those cases where, as described below, the charge transport polymer has a polymerizable functional group at a terminal portion, the structural unit T may be a polymerizable structure (for example, a polymerizable functional group such as a pyrrolyl group).

A specific example of the structural unit T is shown below. However, the structural unit T is not limited to the following structure.

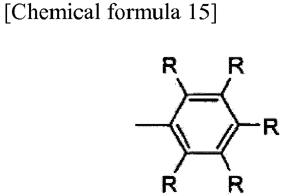

R is the same as R in the structural unit L. In those cases where the charge transport polymer has a polymerizable functional group at a terminal portion, it is preferable that at least one R group is a group containing a polymerizable functional group.

### (Proportions of Structural Units)

From the viewpoint of achieving satisfactory charge transport properties, the proportion of the structural unit L contained in the charge transport polymer, based on the total of all the structural units, is preferably at least 10 mol%, more preferably at least 20 mol%, and even more preferably 30 mol% or higher. If the structural unit T and the optionally introduced structural unit B are also taken into consideration, then the proportion of the structural unit L is preferably not more than 95 mol%, more preferably not more than 90 mol%, and even more preferably 85 mol% or less.

From the viewpoint of improving the characteristics of the organic electronic element, and from the viewpoint of suppressing any increase in viscosity and enabling more favorable synthesis of the charge transport polymer, the proportion of the structural unit T contained in the charge transport polymer, based on the total of all the structural units, is preferably at least 5 mol%, more preferably at least 10 mol%, and even more preferably 15 mol% or higher. Further, from the viewpoint of obtaining satisfactory charge transport properties, the proportion of the structural unit T is preferably not more than 60 mol%, more preferably not more than 55 mol%, and even more preferably 50 mol% or less.

In those cases where the hole transport polymer includes a structural unit B, from the viewpoint of improving the durability of the organic electronic element, the proportion of the structural unit B, based on the total of all the structural units, is preferably at least 1 mol%, more preferably at least 5 mol%, and even more preferably 10 mol% or higher. Further, from the viewpoints of suppressing any increase in viscosity and enabling more favorable synthesis of the charge transport polymer, and from the viewpoint of ensuring satisfactory charge transport properties, the proportion of the structural unit B is preferably not more than 50 mol%, more preferably not more than 40 mol%, and even more preferably 30 mol% or less.

Considering the balance between the charge transport properties, the durability and the productivity and the like, the ratio (molar ratio) between the structural unit L and the structural unit T is preferably L:T = 100:(1 to 70), more preferably 100:(3 to 50), and even more preferably 100:(5 to 30). Further, in those cases where the charge transport polymer also contains a structural unit B, the ratio (molar ratio) between the structural unit L, the structural unit T and the structural unit B is preferably L:T:B = 100:(10 to 200):(10 to 100), more preferably 100:(20 to 180):(20 to 90), and even more preferably 100:(40 to 160):(30 to 80).

The proportion of each structural unit can be determined using the amount added of the monomer corresponding with that structural unit during synthesis of the charge transport polymer. Further, the proportion of each structural unit can also be calculated as an average value using the integral of the spectrum attributable to the structural unit in the ¹H-NMR spectrum of the charge transport polymer. In terms of simplicity, if the amounts added are clear, then the proportion of the structural unit preferably employs the value determined using the amount added of the corresponding monomer.

### (Polymerizable Functional Group)

Further, in order to enable the degree of solubility to be changed, enabling production of a stacked structure of organic thin films, the charge transport compound in the present embodiment preferably has at least one "polymerizable functional group". This "polymerizable functional group" describes a functional group that is able to form bonds between two or more molecules by initiating a polymerization reaction, and details are described below.

Examples of the polymerizable functional group include groups having a carbon-carbon multiple bond (such as a vinyl group, acetylene group, butenyl group, acrylic group, acrylate group, acrylamide group, methacrylic group, methacrylate group, methacrylamide group, arene group, allyl group, vinyl ether group, vinylamino group, furyl group, pyrrole group, thiophene group and silole group), groups having a small ring (including a cyclopropyl group and cyclobutyl group, cyclic ether groups such as an epoxy group and oxetanyl group, diketene groups, and episulfide groups), lactone groups, lactam groups, and groups containing a siloxane derivative. Other examples include heterocyclic groups (such as a furanyl group, pyrrolyl group, thiophenyl group and silolyl group). Further, in addition to the above groups, combinations of groups capable of forming an ester linkage or amide linkage may also be used. Examples include a combination of an ester group and an amino group, or an ester group and a hydroxyl group. From the viewpoint of reactivity, an oxetanyl group, epoxy group, vinyl group, vinyl ether group, acrylate group or methacrylate group is particularly preferred, and an oxetantyl group is the most desirable. From the viewpoints of improving the degree of freedom associated with the polymerizable substituent, and facilitating the curing reaction, the main chain of the polymer or oligomer and the polymerizable functional group are preferably linked via an alkyl chain of 1 to 8 carbon atoms.

Further, in the case where, for example, the organic layer is to be formed on an electrode, from the viewpoint of enhancing the affinity with hydrophilic electrodes of ITO or the like, the main chain and the polymerizable functional group are preferably linked via a hydrophilic chain such as an ethylene glycol chain or a diethylene glycol chain. Moreover, from the viewpoint of simplifying preparation of the monomer used for introducing the polymerizable functional group, the charge transport polymer may have an ether linkage or an ester linkage at the terminal of the alkylene chain and/or the hydrophilic chain, namely, at the linkage site between these chains and the polymerizable functional group, and/or at the linkage site between these chains and the backbone of the charge transport polymer. The "group containing a polymerizable functional group" mentioned above means either the polymerizable functional group itself, or a group composed of a combination of the polymerizable functional group and an alkylene chain or the like. Examples of groups that can be used favorably as this group containing a polymerizable functional group include the groups described in WO 2010/140553.

The polymerizable functional group may be introduced at a terminal portion of the charge transport polymer (namely, a structural unit T), at a portion other than a terminal portion (namely, a structural unit L or B), or at both a terminal portion and a portion other than a terminal. From the viewpoint of the curability, the polymerizable functional group is preferably introduced at least at a terminal portion, and from the viewpoint of achieving a combination of favorable curability and charge transport properties, is preferably introduced only at terminal portions. Further, in those cases where the charge transport polymer has a branched structure, the polymerizable functional group may be introduced within the main chain of the charge transport polymer, within a side chain, or within both the main chain and a side chain.

From the viewpoint of contributing to a change in the solubility, the polymerizable functional group is preferably included in the charge transport polymer in a large amount. On the other hand, from the viewpoint of not impeding the charge transport properties, the amount included in the charge transport polymer is preferably kept small. The amount of the polymerizable functional group may be set as appropriate with due consideration of these factors.

For example, from the viewpoint of obtaining a satisfactory change in the solubility, the number of polymerizable functional groups per one molecule of the charge transport polymer is preferably at least 2, and more preferably 3 or greater. Further, from the viewpoint of maintaining good charge transport properties, the number of polymerizable functional groups is preferably not more than 1,000, and more preferably 500 or fewer.

The number of polymerizable functional groups per one molecule of the charge transport polymer can be determined as an average value from the amount of the polymerizable functional group used in synthesizing the charge transport polymer (for example, the amount added of the monomer having the polymerizable functional group), the amounts added of the monomers corresponding with the various structural units, and the weight average molecular weight of the charge transport polymer and the like. Further, the number of polymerizable functional groups can also be calculated as an average value using the ratio between the integral of the signal attributable to the polymerizable functional group and the integral of the total spectrum in the ¹H-NMR (nuclear magnetic resonance) spectrum of the charge transport polymer, and the weight average molecular weight of the charge transport polymer and the like. In terms of simplicity, if the amounts added of the various components are clear, then the number of polymerizable functional groups is preferably determined from these amounts.

In those cases where the charge transport polymer has a polymerizable functional group, from the viewpoint of ensuring efficient curing of the charge transport polymer, the proportion of the polymerizable functional group, based on the total of all the structural units, is preferably at least 0.1 mol%, more preferably at least 1 mol%, and even more preferably 3 mol% or higher. Further, from the viewpoint of ensuring favorable charge transport properties, the proportion of the polymerizable functional group is preferably not more than 70 mol%, more preferably not more than 60 mol%, and even more preferably 50 mol% or less. Here, the "proportion of the polymerizable functional group" refers to the proportion of structural units having the polymerizable functional group.

### (Production Method)

The charge transport polymer can be produced by various synthesis methods, and there are no particular limitations. For example, conventional coupling reactions such as the Suzuki coupling, Negishi coupling, Sonogashira coupling, Stille coupling and Buchwald-Hartwig coupling reactions can be used. The Suzuki coupling is a reaction in which a cross-coupling reaction is initiated between an aromatic boronic acid derivative and an aromatic halide using a Pd catalyst. By using a Suzuki coupling, the charge transport polymer can be produced easily by bonding together the desired aromatic rings.

In the coupling reaction, a Pd(0) compound, Pd(II) compound, or Ni compound or the like is used as a catalyst. Further, a catalyst species generated by mixing a precursor such as tris(dibenzylideneacetone)dipalladium(0) or palladium(II) acetate with a phosphine ligand can also be used. Reference may also be made to WO 2010/140553 in relation to synthesis methods for the charge transport polymer.

### (Number Average Molecular weight)

Further, in those cases where the charge transport compound described above is a polymer or an oligomer, from the viewpoints of the solubility in solvents and the film formability, the number average molecular weight is preferably at least 500, and is more preferably at least 1,000 but not more than 1,000,000. The number average molecular weight is more preferably at least 2,000 but not more than 900,000, even more preferably at least 3,000 but not more than 800,000, and still more preferably not more than 50,000. If the number average molecular weight is less than 1,000, then crystallization tends to occur more readily, and the film formability deteriorates. Further, if the number average molecular weight exceeds 1,000,000, then the solubility in solvents deteriorates, and producing a coating solution or coating ink becomes problematic.

### (Weight Average Molecular Weight)

Further, in those cases where the charge transport compound described above is a polymer or an oligomer, the weight average molecular weight can be adjusted appropriately with due consideration of the solubility in solvents and the film formability and the like. From the viewpoint of ensuring superior charge transport properties, the weight average molecular weight is preferably at least 1,000, more preferably at least 5,000, and even more preferably 10,000 or greater. Further, from the viewpoints of maintaining favorable solubility in solvents and facilitating the preparation of the ink composition described below, the weight average molecular weight is preferably not more than 1,000,000, more preferably not more than 700,000, and even more preferably 400,000 or less.

The number average molecular weight and the weight average molecular weight can be measured by gel permeation chromatography (GPC), using a calibration curve of standard polystyrenes.

### (Amount)

From the viewpoint of obtaining favorable charge transport properties, the amount of the charge transport compound, relative to the total mass of the organic electronic material, is preferably at least 50% by mass, more preferably at least 70% by mass, and even more preferably 80% by mass or greater. The amount may be 100% by mass.

In one embodiment, a charge transport compound having a deep highest occupied molecular orbital (HOMO) can also be used favorably as the charge transport compound.

In those cases where the organic electronic material is used in a hole injection layer and/or a hole transport layer in an organic EL element, from the viewpoint of reducing the hole injection barrier for injecting a hole from the hole transport layer into a light-emitting layer having a deep HOMO, a charge transport compound having a deep HOMO (a large absolute value for the HOMO) may sometimes be desirable.

On the other hand, tests have also been conducted into improving the charge transport properties of charge transport compounds by adding an electron-accepting compound to the charge transport compound for the purpose of reducing the drive voltage of organic electronic elements such as organic EL elements. However, particularly in those cases where a charge transport compound having a deep highest occupied molecular orbital (HOMO) is used, reducing the voltage of an organic EL element has proven difficult.

In contrast, in the case of the organic electronic material of the present embodiment, by using the ionic compound represented by general formula (1) in combination with the charge transport compound, a reduction in the drive voltage and stable long-term operation can be achieved even when a charge transport compound having a deep HOMO is used. The reasons for this are not entirely clear, but it is thought that because the ionic compound represented by general formula (1) has a specific cation structure containing a fluorinated benzyl group, doping of charge transport compounds having a deep HOMO can occur rapidly, yielding an effect that lowers the drive voltage of the organic EL element. It should be noted that this mechanism is merely a theory, and in no way limits the present invention.

Among the charge transport compounds described above, examples of charge transport compounds having a deep HOMO include compounds having a fluorinated aryl structure or phenylene structure within the molecule. In this description, a charge transport compound having a deep HOMO means a compound for which the HOMO is -5.2 eV or lower, and preferably -5.3 eV or lower. The absolute value of the HOMO is a value measured using the method described in the examples below, and a larger absolute value means a deeper HOMO. Specific examples of charge transport compounds having a deep HOMO include charge transport compounds 2 and 3 described in the examples below.

Further, in order to utilize the difference in the degree of solubility caused by the polymerization reaction, the organic electronic material of the present embodiment preferably also contains a polymerization initiator.

There are no particular limitations on this polymerization initiator, provided it has the ability to initiate polymerization of the polymerizable functional group upon application of heat, light, microwaves, radiation, or an electron beam or the like, but a polymerization initiator that initiates polymerization upon light irradiation and/or heat application is preferred. Further, from the viewpoint of enabling simpler preparation of the ink composition described below, the use of a material that combines both a function as a polymerization initiator and a function as a dopant is preferred.

The ionic compound represented by general formula (1) may be used alone as a polymerization initiator. Further, a combination of the ionic compound represented by general formula (1) and another polymerization initiator may also be used.

### [Other Optional Components]

The organic electronic material may also contain other charge transport compounds besides the charge transport compound described above, as well as other polymers and the like.

### <Ink Composition>

In one embodiment, the organic electronic material described above may also include a solvent capable of dissolving or dispersing the material, thus forming an ink composition. The ink composition contains at least the organic electronic material of the embodiment described above, and a solvent capable of dissolving or dispersing the material. The ink composition may, if necessary, also contain various conventional additives, provided the characteristics achieved by using the organic electronic material are not impaired. For example, various additives may be included, such as polymerization inhibitors, stabilizers, thickeners, gelling agents, flame retardants, antioxidants, reduction inhibitors, oxidizing agents, reducing agents, surface modifiers, emulsifiers, antifoaming agents, dispersants and surfactants. By using this type of ink composition, an organic layer can be formed easily using a simple coating method.

Water, organic solvents, or mixed solvents thereof may be used as the solvent. Examples of the organic solvent include alcohols such as methanol, ethanol and isopropyl alcohol, alkanes such as pentane, hexane and octane, cyclic alkanes such as cyclohexane, aromatic hydrocarbon solvents such as benzene, toluene, xylene, mesitylene, tetralin and diphenylmethane, aliphatic ethers such as ethylene glycol dimethyl ether, ethylene glycol diethyl ether and propylene glycol-1-monomethyl ether acetate, aromatic ethers such as 1,2-dimethoxybenzene, 1,3-dimethoxybenzene, anisole, phenetole, 2-methoxytoluene, 3-methoxytoluene, 4-methoxytoluene, 2,3-dimethylanisole and 2,4-dimethylanisole, aliphatic esters such as ethyl acetate, n-butyl acetate, ethyl lactate and n-butyl lactate, aromatic esters such as phenyl acetate, phenyl propionate, methyl benzoate, ethyl benzoate, propyl benzoate and n-butyl benzoate, amide-based solvents such as N,N-dimethylformamide and N,N-dimethylacetamide, as well as other solvents such as dimethyl sulfoxide, tetrahydrofuran, acetone, chloroform and methylene chloride and the like. Preferred solvents include aromatic hydrocarbon solvents, aliphatic esters, aromatic esters, aliphatic ethers, and aromatic ethers and the like.

In the ink composition of the present embodiment, the amount of the organic electronic material relative to the amount of the solvent may be adjusted to enable use of the ink composition in various coating processes. For example, the amount of the charge transport compound relative to 100% by mass of the solvent is preferably from 0.1 to 30% by mass. This amount is more preferably at least 0.2% by mass, and even more preferably 0.5% by mass or greater, and is more preferably not more than 15% by mass, and even more preferably 10% by mass or less.

### <Organic Layer>

Using the organic electronic material of an embodiment of the present invention, various types of layers used in organic electronic elements and the like can be formed. Although there are no particular limitations on the method used for forming the layer, from the viewpoint of enabling multilayering of organic EL elements to be achieved more easily, film formation using a coating method is preferred.

A typical example of a method for achieving film formation by a coating method includes a coating step in which a solution (ink composition) containing the organic electronic material of an embodiment of the present invention is applied to the desired substrate using a conventional method, for example, a plateless printing method such as an inkjet method, a casting method, a dipping method, a printing method such as relief printing, intaglio printing, offset printing, lithographic printing, relief reversal offset printing, screen printing or gravure printing, or a plate-based printing method such as a spin-coating method, and if necessary a drying step in which the coating layer obtained following application is dried using a hot plate or oven to remove the solvent. In those cases where the charge transport polymer has a polymerizable functional group, film formation can be achieved by subjecting the polymer or oligomer to a polymerization reaction by performing light irradiation or a heat treatment or the like, thereby changing the degree of solubility of (and curing) the organic layer. The substrate may be any of various substrates typically used in organic electronic elements, or may be another previously formed layer. This other previously formed layer may be an organic layer of an embodiment of the present invention. By repeating these types of operations, multilayering of polymer type organic electronic elements and organic EL elements can be achieved.

The above type of coating method (coating step) is typically conducted in a temperature range from -20 to +300°C, preferably from 10 to 100°C, and particularly preferably from 15 to 50°C. Further, although there are no particular limitations on the solvent used in the above solution, one example is the solvent used in preparing the above ink composition.

Furthermore, for the light irradiation, a light source such as a low-pressure mercury lamp, medium-pressure mercury lamp, high-pressure mercury lamp, ultra-high-pressure mercury lamp, metal halide lamp, xenon lamp, fluorescent lamp, light-emitting diode, or sunlight or the like may be used.

Further, the heat treatment mentioned above may be performed on a hot plate or in an oven, and in the present embodiment, because excellent curability is obtained by using the ionic compound described above, (1) curing at low temperature, and (2) curing in a short period of time are possible. Curing at low temperature is useful when using a resin substrate or the like having a low heat-resistant temperature, whereas curing in a short period of time contributes to an improvement in the productivity.

Specifically, in the case of (1) above, the heat treatment may be performed at a heating temperature that is within a range from 0 to 300°C, preferably from 50 to 250°C, and particularly preferably from 50 to 200°C. When curing is performed at low temperature, the heating time is, for example, from 5 to 60 minutes, and preferably from 10 to 40 minutes. In the case of (2) above, the heating time may be from 1 to 60 minutes, and preferably from 1 to 20 minutes. When curing is completed in a short period of time, the heating temperature is typically from 180 to 250°C, and preferably from 200 to 230°C.

From the viewpoint of improving the efficiency of charge transport, the thickness of the organic layer after drying or curing is preferably at least 0.1 nm, more preferably at least 1 nm, and even more preferably 3 nm or greater. Further, from the viewpoint of reducing the electrical resistance, the thickness of the organic layer is preferably not more than 300 nm, and more preferably 200 nm or less.

In organic EL elements, and particularly in polymer type organic EL elements, in terms of improving the emission efficiency and lifespan characteristics, it is desirable that multilayering of the organic layers is performed, and that the functions of the various layers are separated. On the other hand, in order to enable multilayering of the organic layers to be performed by film formation using wet processes such as printing or inkjet techniques, which enable film formation to be performed across even large surface areas, it is necessary to ensure that the lower layer does not dissolve during formation of the upper layer. The method disclosed in the aforementioned Patent Document 1 that uses a difference in the degree of solubility is an effective technique for enabling multilayering of organic layers, but suffers from a problem in that if water-soluble PEDOT:PSS is used, then residual moisture within the thin film must be removed. Further, the method disclosed in the aforementioned Patent Document 2 in which a polymerization reaction is used to change the degree of solubility in solvents also has room for improvement, not only because the variety of materials that can be used is limited, but from the viewpoints of the stability relative to moisture in the air and the characteristics of the resulting element.

Further, ink compositions containing the above materials either require treatment at high temperature to achieve curing, meaning application to resin substrates is problematic, or require heating to be performed for a long period of time, meaning the productivity is poor.

However, the organic electronic material according to one embodiment of the present invention exhibits excellent curability, and particularly superior curability at low temperatures, and therefore even in those cases where a resin substrate or the like is used, an organic electronic element having multilayered organic layers can be produced at high yield.

### <Organic Electronic Element, Organic Electroluminescent Element>

An organic electronic element of an embodiment of the present invention preferably contains an organic layer formed using either the organic electronic material described above or the ink composition described above, and more preferably contains a layer that has been insolubilized by polymerizing the formed layer. The organic layer formed using the organic electronic material or the ink composition is preferably formed by a coating method.

In a similar manner, an organic electroluminescent element (organic EL element) of an embodiment of the present invention preferably contains an organic layer formed using either the organic electronic material described above or the ink composition described above, and more preferably contains a layer that has been insolubilized by polymerizing the formed layer.

Both of these elements contain a layer having excellent charge transport properties that has been formed using the organic electronic material of an embodiment of the present invention, and have a low drive voltage and a long emission lifespan.

The organic electronic element of an embodiment of the present invention can be produced by a method that includes a step of forming an organic layer using the aforementioned organic electronic material or ink composition, for example by using a coating method.

An organic EL element of an embodiment of the present invention is described below in detail.

### [Organic EL Element]

There are no particular limitations on the organic EL element of the present embodiment, provided it contains a light-emitting layer, an anode, a cathode and a substrate, but the element may also contain other layers such as a hole injection layer, electron injection layer, hole transport layer or electron transport layer. Each layer may be formed by a vapor deposition method or by a coating method. Further, it is preferable that at least one of a hole injection layer, a hole transport layer and the light-emitting layer is formed using the organic electronic material or the ink composition of an embodiment of the present invention, and it is more preferable that at least one of a hole injection layer and a hole transport layer is formed using the organic electronic material or the ink composition of an embodiment of the present invention. In one embodiment, formation of this organic layer can be performed favorably by a coating method using the previously described ink composition.

FIG. 1 is a cross-sectional schematic view illustrating one example of an organic EL element according to an embodiment of the present invention. The organic EL element in FIG. 1 is an element having a multilayer structure, and includes a substrate 8, an anode 2, a hole injection layer 3 and a hole transport layer 6 formed from an organic layer of the embodiment described above, a light-emitting layer 1, an electron transport layer 7, an electron injection layer 5 and a cathode 4 formed in that order. Each of these layers is described below.

### (Light-Emitting Layer)

Examples of materials that can be used for the light-emitting layer include low-molecular weight compounds, polymers or oligomers, and dendrimers and the like. Polymers exhibit good solubility in solvents, meaning they are suitable for coating methods, and are consequently preferred. Examples of the light-emitting material include fluorescent materials, phosphorescent materials, and thermally activated delayed fluorescent materials (TADF).

Examples of low-molecular weight compounds that use fluorescence emission include perylene, coumarin, rubrene, quinacridone, color laser dyes (such as rhodamine and DCM1), aluminum complexes (such as tris(8-hydroxyquinolinato)aluminum(III) (Alq₃)), stilbene, and derivatives of these compounds. Examples of polymers or oligomers using fluorescence emission that can be used favorably include polyfluorene, polyphenylene, polyphenylenevinylene (PPV), polyvinylcarbazole (PVK), fluorene-benzothiadiazole copolymers, fluorene-triphenylamine copolymers, and derivatives and mixtures of these compounds.

On the other hand, in recent years, in order to further improve the efficiency of organic EL elements, phosphorescent organic EL elements are also being actively developed. In a phosphorescent organic EL element, not only singlet state energy, but also triplet state energy can be used, and therefore the internal quantum yield can, in principle, be increased to 100%. In a phosphorescent organic EL element, a metal complex-based phosphorescent material containing a heavy metal such as platinum or iridium is used as a phosphorescence-emitting dopant for doping a host material, thus enabling the extraction of a phosphorescence emission (see M.A. Baldo et al., Nature, vol. 395, p. 151 (1998), M.A. Baldo et al., Applied Physics Letters, vol. 75, p. 4 (1999), M.A. Baldo et al., Nature, vol. 403, p. 750 (2000)).

In the organic EL element of the present embodiment, from the viewpoint of improving the element efficiency, a phosphorescent material is preferably used for the light-emitting layer. Examples of materials that can be used favorably as the phosphorescent material include metal complexes and the like containing Ir or Pt or the like as a central metal. Specific examples of Ir complexes include FIr(pic) (iridium(III) bis[(4,6-difluorophenyl)-pyridinato-N,C²]picolinate) which emits blue light, Ir(ppy)₃ (fac-tris(2-phenylpyridine)iridium) which emits green light (see M.A. Baldo et al., Nature, vol. 403, p. 750 (2000)), and (btp)₂Ir(acac) {bis[2-(2'-benzo[4,5-α]thienyl)pyridinato-N,C³]iridium(acetyl-acetonate)} and Ir(piq)₃ (tris(1-phenylisoquinoline)iridium) which emit red light and are disclosed in Adachi et al., Appl. Phys. Lett., 78 No. 11, 2001, 1622.

Specific examples of Pt complexes include platinum 2,3,7,8,12,13,17,18-octaethyl-21H,23H-porphyrin (PtOEP) which emits red light.

The phosphorescent material can use a low-molecular weight compound or a dendrite such as an iridium core dendrimer. Derivatives of these compounds can also be used favorably.

Further, when a phosphorescent material is incorporated in the light-emitting layer, a host material is preferably included in addition to the phosphorescent material.

The host material may be a low-molecular weight compound, a high-molecular weight compound, or a dendrimer or the like. The organic electronic material of an embodiment of the present invention may also be used as the host material.

Examples of low-molecular weight compounds that can be used include CBP (4,4'-bis(carbazol-9-yl)-biphenyl), mCP (1,3-bis(9-carbazolyl)benzene), CDBP (4,4'-bis(carbazol-9-yl)-2,2'-dimethylbiphenyl), and derivatives of these compounds, whereas examples of high-molecular weight compounds that can be used include polyvinylcarbazole, polyphenylene and polyfluorene, and derivatives of these compounds.

Examples of the thermally activated delayed fluorescent materials include the compounds disclosed in Adv. Mater., 21, 4802-4906 (2009); Appl. Phys. Lett., 98, 083302 (2011); Chem. Comm., 48, 9580 (2012); Appl. Phys. Lett., 101, 093306 (2012); J. Am. Chem. Soc., 134, 14706 (2012); Chem. Comm., 48, 11392 (2012); Nature, 492, 234 (2012); Adv. Mater., 25, 3319 (2013); J. Phys. Chem. A, 117, 5607 (2013); Phys. Chem. Chem. Phys., 15, 15850 (2013); Chem. Comm., 49, 10385 (2013); and Chem. Lett., 43, 319 (2014) and the like.

The light-emitting layer may be formed by a vapor deposition method or a coating method.

Forming the light-emitting layer by a coating method enables the organic EL element to be produced more cheaply, and is consequently preferred. Formation of the light-emitting layer by a coating method can be achieved by using a conventional coating method such as an inkjet method, casting method, dipping method, a printing method such as relief printing, intaglio printing, offset printing, lithographic printing, relief reversal offset printing, screen printing or gravure printing, or a spin-coating method to apply a solution containing the phosphorescent material, and if necessary a host material, to a desired substrate.

### (Cathode)

The cathode material is preferably a metal or a metal alloy, such as Li, Ca, Mg, Al, In, Cs, Ba, Mg/Ag, LiF or CsF.

### (Anode)

A metal (for example, Au) or another material having metal-like conductivity such as an oxide (for example, indium tin oxide (ITO)) or a conductive polymer (for example, polythiophene-polystyrene sulfonate mixtures (PEDOT:PSS)) may be used as the cathode.

### (Electron Transport Layer, Electron Injection Layer)

Examples of materials for the electron transport layer and electron injection layer include phenanthroline derivatives (such as 2,9-dimethyl-4,7-diphenyl-1,10-phenanthroline (BCP)), bipyridine derivatives, nitro-substituted fluorene derivatives, diphenylquinone derivatives, thiopyran dioxide derivatives, heterocyclic tetracarboxylic acid anhydrides of naphthalene and perylene and the like, carbodiimides, fluorenylidenemethane derivatives, anthraquinodimethane and anthrone derivatives, oxadiazole derivatives (such as 2-(4-biphenylyl)-5-(4-tert-butylphenyl-1,3,4-oxadiazole (PBD)), benzimidazole derivatives, and aluminum complexes (such as tris(8-hydroxyquinolinato)aluminum(III) (Alq₃)). Moreover, thiadiazole derivatives in which the oxygen atom in the oxadiazole ring of the oxadiazole derivatives mentioned above has been substituted with a sulfur atom, and quinoxaline derivatives having a quinoxaline ring that is well known as an electron-withdrawing group can also be used. Furthermore, the organic electronic material of an embodiment of the present invention may also be used.

### (Hole Injection Layer and Hole Transport Layer)

The organic EL element of an embodiment of the present invention preferably uses an organic electronic material of an embodiment of the present invention, containing the ionic compound represented by general formula (1) and a charge transport compound, for at least one of a hole injection layer and a hole transport layer. The organic electronic material of the present invention may be used for one of the hole injection layer and the hole transport layer, with another material being used for the other layer.

Examples of materials that may be used for a hole injection layer or a hole transport layer, besides the materials described in the present description, include, but are not limited to, aromatic amine-based compounds (for example, aromatic diamines such as α-NPD), phthalocyanine-based compounds, and thiophene compounds (for example, thiophene-based conductive polymer:poly(4-styrenesulfonate)) (PEDOT:PSS)).

### (Substrate)

In terms of substrates that can be used in the organic EL element of the present embodiment, various types of glass and plastic may be used without any particular restrictions, and a transparent substrate is preferred. Glass, quartz and light-transmitting resin films and the like can be used favorably, but the substrate is not limited to these materials. If a resin film (flexible substrate) is used, then the organic EL element can also be imparted with flexibility, which is particularly desirable. Further, because the organic electronic material of an embodiment of the present invention exhibits excellent low-temperature curability, it can be used particularly favorably with organic electronic elements that use a resin film as the substrate.

Examples of the resin films include films formed from polyethylene terephthalate (PET), polyethylene naphthalate (PEN), polyethersulfone (PES), polyetherimide, polyetheretherketone, polyphenylene sulfide, polyarylate, polyimide, polycarbonate (PC), cellulose triacetate (TAC), and cellulose acetate propionate (CAP) and the like.

Furthermore, in those cases when a resin film is used, an inorganic substance such as silicon oxide or silicon nitride may be coated onto the resin film to inhibit the transmission of water vapor and oxygen and the like.

### (Emission Color)

Although there are no particular limitations on the color of the light emission from the organic EL element of the present embodiment, white light-emitting elements can be used for various lighting fixtures, including domestic lighting, in-vehicle lighting, watches and liquid crystal backlights, and are consequently preferred.

Because generating white light emission from a single material is currently impossible, the method used for forming a white light-emitting element involves using a plurality of light-emitting materials to emit a plurality of colors simultaneously, and then mixing the emitted colors to obtain a white light emission. There are no particular limitations on the combination of the plurality of emission colors, and examples include combinations that include three maximum emission wavelengths for blue, green and red, and combinations that include two maximum emission wavelengths for blue and yellow, or yellowish green and orange. Further, control of the emission color can be achieved by appropriate adjustment of the type and amount of the phosphorescent material.

### <Display Element, Illumination Device, Display Device>

A display element of an embodiment of the present invention includes the organic EL element of the embodiment described above.

For example, by using the organic EL element of the above embodiment as the element corresponding with each color pixel of red, green and blue (RGB), a color display element can be obtained.

Image formation may employ a simple matrix in which organic EL elements arrayed in a panel are driven directly by an electrode arranged in a matrix, or an active matrix in which a thin-film transistor is positioned on, and drives, each element. The former has a simpler structure, but there is a limit to the number of vertical pixels, and therefore these types of displays are typically used for displaying text or the like. The latter has a lower drive voltage, requires less current and yields a bright high-quality image, and is therefore typically used for high-quality displays.

Further, an illumination device of an embodiment of the present invention includes the organic EL element of the embodiment described above. Moreover, a display device of an embodiment of the present invention includes the illumination device and a liquid crystal element as a display unit. A display device that uses the illumination device of an embodiment of the present invention as a backlight (white light-emitting source) and uses a liquid crystal element as the display unit, namely a liquid crystal display device, is also possible. This configuration is merely a conventional liquid crystal display device in which only the backlight has been replaced with the illumination device of an embodiment of the present invention, with the liquid crystal element portion employing conventional technology.

The organic electronic material according to an embodiment of the present invention exhibits excellent charge transport properties, and can therefore be used favorably not only in organic EL elements, but also in various other organic electronic elements.

### EXAMPLES

The present invention is described below in further detail using a series of examples, but the present invention is in no way limited by the following examples.

### <1> Synthesis of Ionic Compounds

### [Ionic Compound 1]

A three-neck round-bottom flask was charged with toluene (20 ml), 2.8 g (0.02 mol) of 4-fluoro-N-methylbenzylamine, 7.6 g (0.04 mol) of 4-fluorobenzyl bromide, 7.9 g of an aqueous solution (50%) of sodium hydroxide, and 0.64 g (0.002 mol) of tetrabutylammonium bromide, and the mixture was heated and stirred under reflux for 10 hours. Following completion of the stirring, the organic layer was washed with water, 5 g of magnesium sulfate was added to dewater the organic layer, and the organic layer was then concentrated using a rotary evaporator. Subsequently, silica gel column chromatography (eluent = hexane:ethyl acetate = 1:1 (volume ratio)) was used to obtain N-(4-fluorobenzyl)-1-(4-fluorophenyl)-N-methylmethanamine (I) (yield: 4.0 g, reaction yield: 80%).

Next, 1.7 g of hydrobromic acid (48%) was mixed with 2.5 g (0.01 mol) of the above N-(4-fluorobenzyl)-1-(4-fluorophenyl)-N-methylmethanamine (I), the mixture was heated slightly and shaken, and after subsequently standing for one hour, the water content was removed under reduced pressure to obtain a viscous oily substance. Analysis of this oily substance by proton NMR confirmed that the N-(4-fluorobenzyl)-1-(4-fluorophenyl)-N-methylmethanamine (I) had disappeared, and N-(4-fluorobenzyl)-1-(4-fluorophenyl)-N-methylmethanammonium bromide (II) had been produced.

Subsequently, 1.6 g (0.005 mol) of the above compound (II) and 35.1 g (0.005 mol) of sodium tetrakis(pentafluorophenyl)borate (10% aq.) were mixed and stirred. When the thus obtained reaction mixture was left to stand overnight, a white precipitate developed in an overall cloudy white jelly. An appropriate amount of water was added, the mixture was filtered under reduced pressure, and the filtered precipitate was washed with water and dried to obtain a white solid (yield: 3.5 g, reaction yield: 75%).

The reaction equation for the above reactions is shown below.

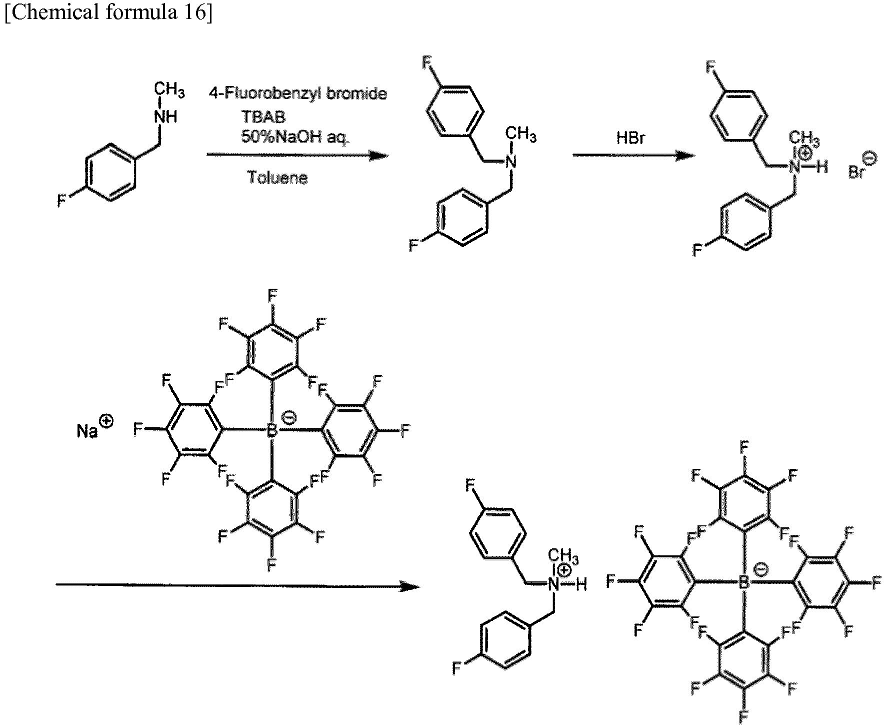

### [Ionic Compound 2]

A three-neck round-bottom flask was charged with toluene (20 ml), 2.8 g (0.02 mol) of 4-fluoro-N-methylbenzylamine, 6.9 g (0.04 mol) of benzyl bromide, 7.9 g of an aqueous solution (50%) of sodium hydroxide, and 0.64 g (0.002 mol) of tetrabutylammonium bromide, and the mixture was heated and stirred under reflux for 10 hours. Following completion of the stirring, the organic layer was washed with water, 5 g of magnesium sulfate was added to dewater the organic layer, and the organic layer was then concentrated using a rotary evaporator. Subsequently, silica gel column chromatography (eluent = hexane:ethyl acetate = 1:1 (volume ratio)) was used to obtain N-benzyl-1-(4-fluorophenyl)-N-methylmethanamine (I) (yield: 4.1 g, reaction yield: 90%).

Next, 1.7 g of hydrobromic acid (48%) was mixed with 2.3 g (0.01 mol) of the above N-benzyl-1-(4-fluorophenyl)-N-methylmethanamine (I), the mixture was heated slightly and shaken, and after subsequently standing for one hour, the water content was removed under reduced pressure to obtain a viscous oily substance. Analysis of this oily substance by proton NMR confirmed that the N-benzyl-1-(4-fluorophenyl)-N-methylmethanamine (I) had disappeared, and N-benzyl-1-(4-fluorophenyl)-N-methylmethanammonium bromide (II) had been produced.

Subsequently, 1.6 g (0.005 mol) of the above compound (II) and 35.1 g (0.005 mol) of sodium tetrakis(pentafluorophenyl)borate (10% aq.) were mixed and stirred. When the thus obtained reaction mixture was left to stand overnight, a white precipitate developed in an overall cloudy white jelly. An appropriate amount of water was added, the mixture was filtered under reduced pressure, and the filtered precipitate was washed with water and dried to obtain a white solid (yield: 3.2 g, reaction yield: 70%).

The reaction equation for the above reactions is shown below.

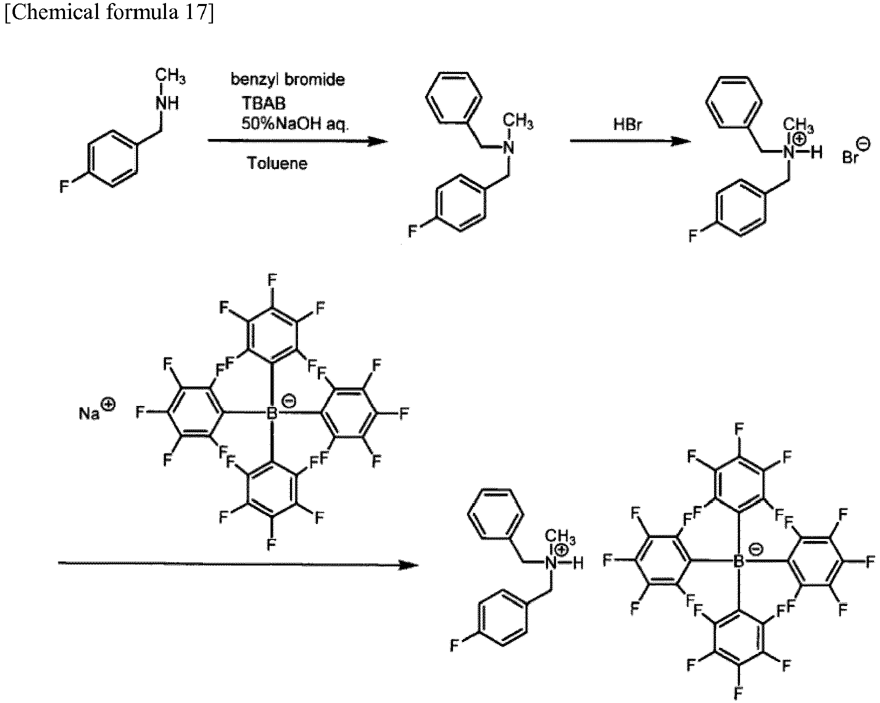

### [Ionic Compound 3]

A three-neck round-bottom flask was charged with toluene (20 ml), 2.8 g (0.02 mol) of 4-fluoro-N-methylbenzylamine, 7.3 g (0.04 mol) of 1-iodobutane, 7.9 g of an aqueous solution (50%) of sodium hydroxide, and 0.64 g (0.002 mol) of tetrabutylammonium bromide, and the mixture was heated and stirred under reflux for 10 hours. Following completion of the stirring, the organic layer was washed with water, 5 g of magnesium sulfate was added to dewater the organic layer, and the organic layer was then concentrated using a rotary evaporator. Subsequently, silica gel column chromatography (eluent = hexane:ethyl acetate = 1:1 (volume ratio)) was used to obtain N-(4-fluorobenzyl)-N-methylbutan-1-amine (I) (yield: 3.5 g, reaction yield: 90%).

Next, 1.7 g of hydrobromic acid (48%) was mixed with 2.0 g (0.01 mol) of the above N-(4-fluorobenzyl)-N-methylbutan-1-amine (I), the mixture was heated slightly and shaken, and after subsequently standing for one hour, the water content was removed under reduced pressure to obtain a viscous oily substance. Analysis of this oily substance by proton NMR confirmed that the N-(4-fluorobenzyl)-N-methylbutan-1-amine (I) had disappeared, and N-(4-fluorobenzyl)-N-methylbutan-1-ammonium bromide (II) had been produced.

Subsequently, 1.38 g (0.005 mol) of the above compound (II) and 35.1 g (0.005 mol) of sodium tetrakis(pentafluorophenyl)borate (10% aq.) were mixed and stirred. When the thus obtained reaction mixture was left to stand overnight, a white precipitate developed in an overall cloudy white jelly. An appropriate amount of water was added, the mixture was filtered under reduced pressure, and the filtered precipitate was washed with water and dried to obtain a white solid (yield: 3.3 g, reaction yield: 75%).

The reaction equation for the above reactions is shown below.

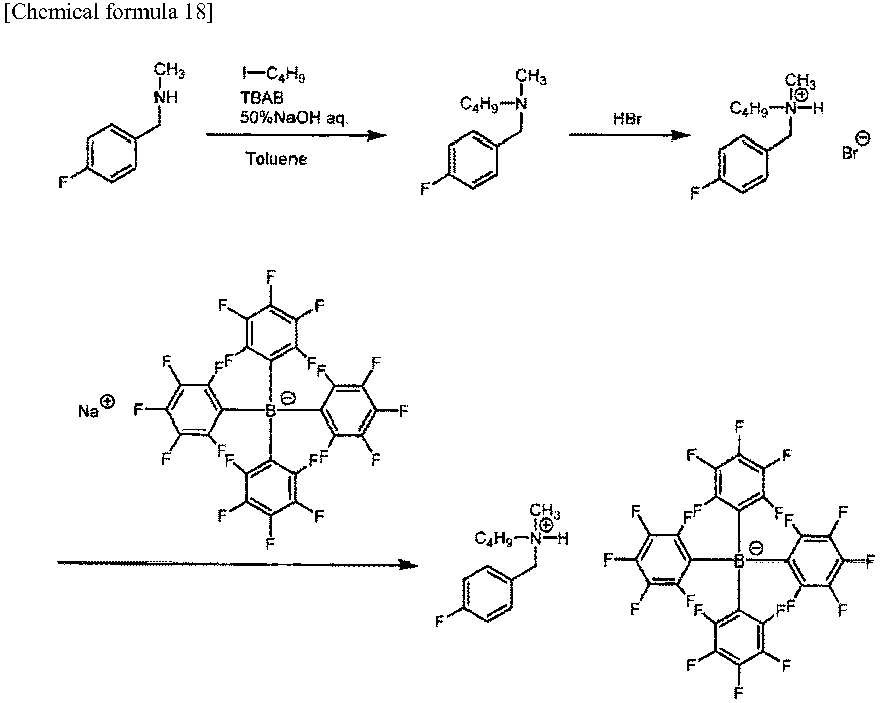

### [Ionic Compound 4]

A three-neck round-bottom flask was charged with toluene (20 ml), 2.8 g (0.02 mol) of 4-fluoro-N-methylbenzylamine, 22.9 g (0.04 mol) of 1-iododecane, 7.9 g of an aqueous solution (50%) of sodium hydroxide, and 0.64 g (0.002 mol) of tetrabutylammonium bromide, and the mixture was heated and stirred under reflux for 10 hours. Following completion of the stirring, the organic layer was washed with water, 5 g of magnesium sulfate was added to dewater the organic layer, and the organic layer was then concentrated using a rotary evaporator. Subsequently, silica gel column chromatography (eluent = hexane:ethyl acetate = 1:1 (volume ratio)) was used to obtain N-(4-fluorobenzyl)-N-methyldecane-1-amine (I) (yield: 3.8 g, reaction yield: 80%).

Next, 1.7 g of hydrobromic acid (48%) was mixed with 2.8 g (0.01 mol) of the above N-(4-fluorobenzyl)-N-methyldecane-1-amine (I), the mixture was heated slightly and shaken, and after subsequently standing for one hour, the water content was removed under reduced pressure to obtain a viscous oily substance. Analysis of this oily substance by proton NMR confirmed that the N-(4-fluorobenzyl)-N-methyldecane-1-amine (I) had disappeared, and N-(4-fluorobenzyl)-N-methyldecane-1-ammonium bromide (II) had been produced.

Subsequently, 1.80 g (0.005 mol) of the above compound (II) and 35.1 g (0.005 mol) of sodium tetrakis(pentafluorophenyl)borate (10% aq.) were mixed and stirred. When the thus obtained reaction mixture was left to stand overnight, a white precipitate developed in an overall cloudy white jelly. An appropriate amount of water was added, the mixture was filtered under reduced pressure, and the filtered precipitate was washed with water and dried to obtain a white solid (yield: 2.4 g, reaction yield: 50%).

The reaction equation for the above reactions is shown below.

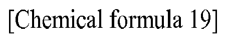

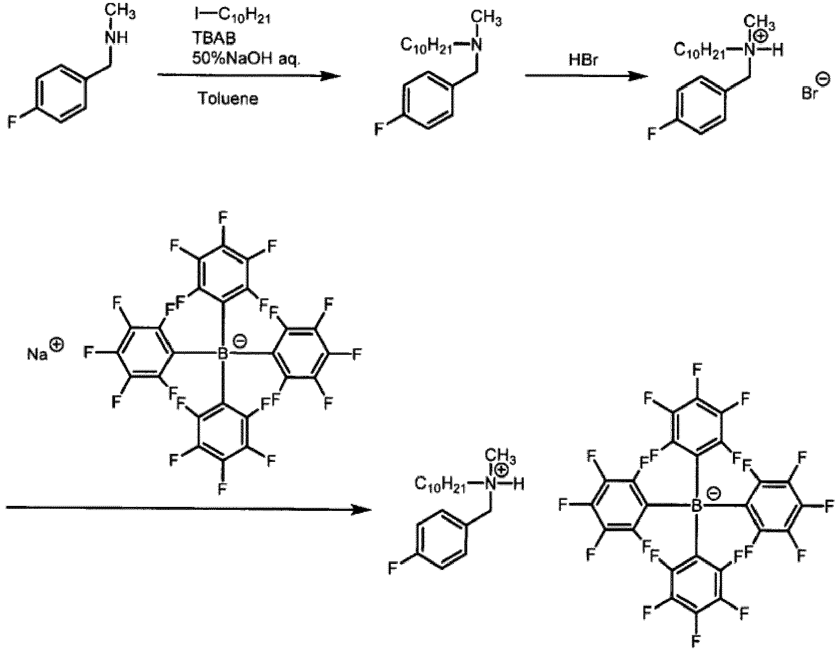

### [Ionic Compound 5]

First, 1.7 g of hydrobromic acid (48%) was mixed with 2.7 g (0.01 mol) of trihexylamine (I), the mixture was heated slightly and shaken, and after subsequently standing for one hour, the water content was removed under reduced pressure to obtain a viscous oily substance. Analysis of this oily substance by proton NMR confirmed that the trihexylamine (I) had disappeared, and trihexylammonium bromide (II) had been produced.

Subsequently, 1.75 g (0.005 mol) of the above compound (II) and 35.1 g (0.005 mol) of sodium tetrakis(pentafluorophenyl)borate (10% aq.) were mixed and stirred. When the thus obtained reaction mixture was left to stand overnight, a white precipitate developed in an overall cloudy white jelly. An appropriate amount of water was added, the mixture was filtered under reduced pressure, and the filtered precipitate was washed with water and dried to obtain a white solid (yield: 2.8 g, reaction yield: 60%).

The reaction equation for the above reactions is shown below.

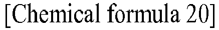

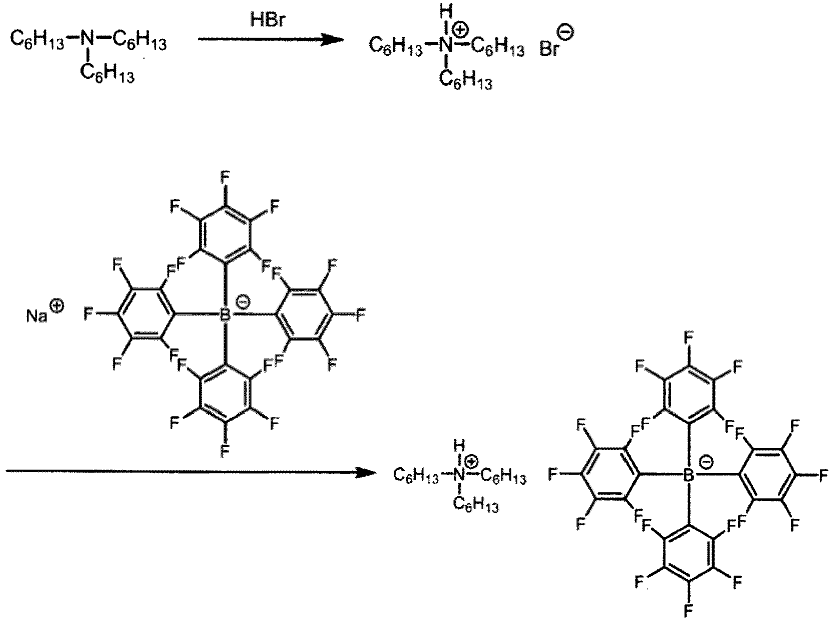

### <2> Synthesis of Charge Transport Compounds

### [Preparation of Pd Catalyst]

In a glove box under a nitrogen atmosphere at room temperature, tris(dibenzylideneacetone)dipalladium (73.2 mg, 80 µmol) was weighed into a sample tube, anisole (15 ml) was added, and the resulting mixture was agitated for 30 minutes. In a similar manner, tris(t-butyl)phosphine (129.6 mg, 640 µmol) was weighed into a sample tube, anisole (5 ml) was added, and the resulting mixture was agitated for 5 minutes. The two solutions were then mixed together and stirred for 30 minutes at room temperature to obtain a catalyst.

### [Charge Transport Compound 1]

A charge transport compound (charge transport polymer) 1 was prepared in the following manner. A three-neck round-bottom flask was charged with a monomer 1 shown below (2.0 mmol), a monomer 2 shown below (5.0 mmol), a monomer 3 shown below (4.0 mmol) and anisole (20 mL), and a solution of the separately prepared Pd catalyst (7.5 mL) was then added and stirred. After stirring for 30 minutes, a 10% aqueous solution of tetraethylammonium hydroxide (20 mL) was added. The resulting mixture was heated and refluxed for 2 hours. All the operations up to this point were conducted under a stream of nitrogen. Further, all of the solvents were deaerated by nitrogen bubbling for at least 30 minutes prior to use.

After completion of the reaction, the organic layer was washed with water, and the organic layer was then poured into methanol-water (9:1 (volume ratio)). The resulting precipitate was collected by filtration under reduced pressure, and then washed with methanol-water (9:1 (volume ratio)). The thus obtained precipitate was dissolved in toluene and re-precipitated from methanol. The obtained precipitate was collected by filtration under reduced pressure and dissolved in toluene, and a metal adsorbent ("Triphenylphosphine, polymer-bound on styrene-divinylbenzene copolymer", manufactured by Strem Chemicals Inc., 200 mg per 100 mg of the precipitate) was then added to the solution and stirred overnight.

Following completion of the overnight stirring, the metal adsorbent and other insoluble matter were removed by filtration, and the filtrate was concentrated using a rotary evaporator. The concentrate was dissolved in toluene, and then re-precipitated from methanol-acetone (8:3 (volume ratio)). The thus produced precipitate was collected by filtration under reduced pressure and washed with methanol-acetone (8:3 (volume ratio)). The resulting precipitate was then dried under vacuum to obtain a charge transport compound 1. The obtained charge transport compound 1 had a number average molecular of 5,600 and a weight average molecular weight of 9,000.

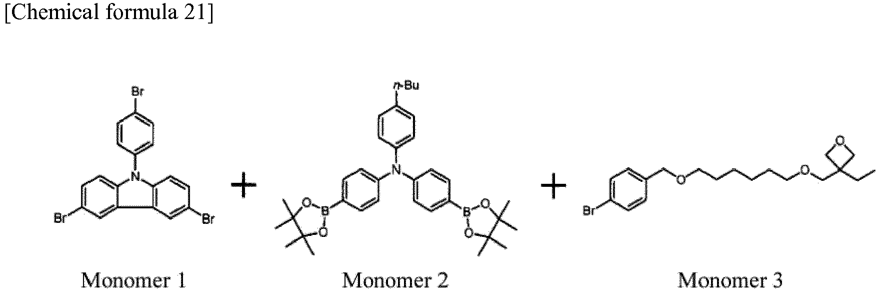

The number average molecular weight and the weight average molecular weight were measured by GPC (relative to polystyrene standards) using tetrahydrofuran (THF) as the eluent. The measurement conditions were as follows.
Feed pump: L-6050, manufactured by Hitachi High-Technologies Corporation
UV-Vis detector: L-3000, manufactured by Hitachi High-Technologies Corporation
Columns: Gelpack (a registered trademark) GL-A160S / GL-A150S, manufactured by Hitachi Chemical Co., Ltd.
Eluent: THF (for HPLC, stabilizer-free), manufactured by Wako Pure Chemical Industries, Ltd.
Flow rate: 1 mL/min
Column temperature: room temperature
Molecular weight standards: standard polystyrenes

### [Charge Transport Compound 2]

With the exception of replacing the monomer 1 with a monomer 4, operations were performed in the same manner as described for the charge transport compound 1, thus obtaining a charge transport compound 2. The obtained charge transport compound 2 had a number average molecular of 5,000 and a weight average molecular weight of 8,000.

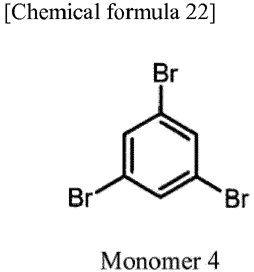

### [Charge Transport Compound 3]

With the exception of replacing the monomer 1 with a monomer 5, operations were performed in the same manner as described for the charge transport compound 1, thus obtaining a charge transport compound 3. The obtained charge transport compound 3 had a number average molecular of 6,000 and a weight average molecular weight of 9,000.

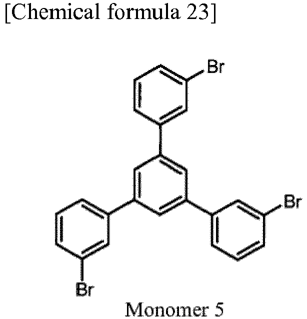

### [Charge Transport Compound 4]

With the exception of replacing the monomer 1 with a monomer 6, operations were performed in the same manner as described for the charge transport compound 1, thus obtaining a charge transport compound 4. The obtained charge transport compound 4 had a number average molecular of 6,000 and a weight average molecular weight of 9,000.

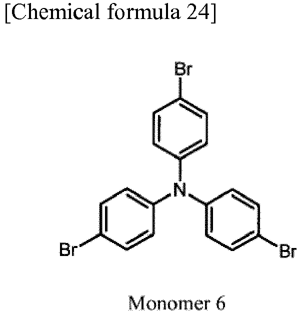

### <3> Measurement of Highest Occupied Molecular Orbital (HOMO) Level of Charge Transport Compounds

### (Measurement of Charge Transport Compound 1)

The charge transport compound 1 (10.0 mg) was dissolved in a toluene (1,000 µl) to prepare an ink composition. This ink composition was spin-coated at 3,000 rpm onto a quartz plate. A heat treatment was then performed on a hot plate at 120°C for 10 minutes, thus obtaining a measurement sample. Subsequently, an AC-5 device (manufactured by Riken Keiki Co., Ltd.) was used to measure the HOMO level of the measurement sample. The measured value was 5.10 eV.

### (Measurement of Charge Transport Compound 2)

With the exception of replacing the charge transport compound 1 with the charge transport compound 2, a measurement was performed in the same manner as described above. The measured value was 5.35 eV.

### (Measurement of Charge Transport Compound 3)

With the exception of replacing the charge transport compound 1 with the charge transport compound 3, a measurement was performed in the same manner as described above. The measured value was 5.45 eV.

### <4> Evaluation of Organic Electronic Materials (Ink Compositions)

### [Example 1]

An ink composition was prepared, and evaluations of the curability and charge transport properties were performed, in the manner described below.

### (Evaluation of Curability)

The charge transport compound 1 (10.0 mg) and the ionic compound 1 (0.15 mg) were dissolved in a toluene (1,000 µl) to prepare an ink composition. This ink composition was spin-coated at 3,000 rpm onto a quartz plate. Heating was then performed on a hot plate at 120°C for 10 minutes to achieve a polymerization reaction. Following heating, the quartz plate was washed by dipping in toluene for one minute. The absorbance (Abs) at the absorption maximum (λmax) in the UV-vis spectrum was measured before and after the washing, and the residual film ratio was determined from the ratio between the two absorbance values. The measurement result is shown in Table 1.

### (Evaluation of Charge Transport Properties)

In order to evaluate the charge transport properties, an evaluation element was produced in the manner described below.

### <Production of Charge Transport Properties Evaluation Element>

The charge transport compound 1 (100 mg), the ionic compound 1 (3.0 mg) and toluene (1.91 mL) were mixed to prepare an ink composition. The prepared ink composition was spin-coated at 3,000 min⁻¹ onto a glass substrate on which ITO had been patterned with a width of 1.6 mm, and the glass substrate was then heated on a hot plate at 120°C for 10 minutes, thus forming a charge transport film (150 nm). The glass substrate having the charge transport film was then transferred into a vacuum deposition apparatus, and aluminum (film thickness: 100 nm) was deposited.

Following deposition of the aluminum, the substrate was transferred under a dry nitrogen atmosphere without exposure to the external atmosphere, and an encapsulating glass having a countersink with a depth of 0.4 mm formed in a 0.7 mm alkali-free glass and the ITO substrate were bonded together using a photocurable epoxy resin, thereby encapsulating and completing preparation of a charge transport properties evaluation element.

A voltage was applied to the charge transport properties evaluation element using the ITO as the anode and the aluminum as the cathode. The applied voltage at a current density of 50 mA/cm² is shown in Table 1.

### [Example 2]

With the exception of replacing the ionic compound 1 from Example 1 with the ionic compound 2, ink compositions were prepared, and the curability and charge transport properties were evaluated, in the same manner as Example 1. The evaluation results are shown in Table 1.

### [Example 3]

With the exception of replacing the ionic compound 1 from Example 1 with the ionic compound 3, ink compositions were prepared, and the curability and charge transport properties were evaluated, in the same manner as Example 1. The evaluation results are shown in Table 1.

### [Example 4]

With the exception of replacing the ionic compound 1 from Example 1 with the ionic compound 4, ink compositions were prepared, and the curability and charge transport properties were evaluated, in the same manner as Example 1. The evaluation results are shown in Table 1.

### [Example 5]

With the exception of replacing the charge transport compound 1 from Example 1 with the charge transport compound 2, ink compositions were prepared, and the curability and charge transport properties were evaluated, in the same manner as Example 1. The evaluation results are shown in Table 1.

### [Example 6]

With the exceptions of replacing the charge transport compound 1 from Example 1 with the charge transport compound 2, and replacing the ionic compound 1 with the ionic compound 2, ink compositions were prepared, and the curability and charge transport properties were evaluated, in the same manner as Example 1. The evaluation results are shown in Table 1.

### [Example 7]

With the exceptions of replacing the charge transport compound 1 from Example 1 with the charge transport compound 2, and replacing the ionic compound 1 with the ionic compound 3, ink compositions were prepared, and the curability and charge transport properties were evaluated, in the same manner as Example 1. The evaluation results are shown in Table 1.

### [Example 8]

With the exceptions of replacing the charge transport compound 1 from Example 1 with the charge transport compound 2, and replacing the ionic compound 1 with the ionic compound 4, ink compositions were prepared, and the curability and charge transport properties were evaluated, in the same manner as Example 1. The evaluation results are shown in Table 1.

### [Example 9]

With the exception of replacing the charge transport compound 1 from Example 1 with the charge transport compound 3, ink compositions were prepared, and the curability and charge transport properties were evaluated, in the same manner as Example 1. The evaluation results are shown in Table 1.

### [Example 10]

With the exceptions of replacing the charge transport compound 1 from Example 1 with the charge transport compound 3, and replacing the ionic compound 1 with the ionic compound 2, ink compositions were prepared, and the curability and charge transport properties were evaluated, in the same manner as Example 1. The evaluation results are shown in Table 1.

### [Example 11]

With the exceptions of replacing the charge transport compound 1 from Example 1 with the charge transport compound 3, and replacing the ionic compound 1 with the ionic compound 3, ink compositions were prepared, and the curability and charge transport properties were evaluated, in the same manner as Example 1. The evaluation results are shown in Table 1.

### [Example 12]

With the exceptions of replacing the charge transport compound 1 from Example 1 with the charge transport compound 3, and replacing the ionic compound 1 with the ionic compound 4, ink compositions were prepared, and the curability and charge transport properties were evaluated, in the same manner as Example 1. The evaluation results are shown in Table 1.

### [Comparative Example 1]

With the exception of replacing the ionic compound 1 from Example 1 with the ionic compound 5, ink compositions were prepared, and the curability and charge transport properties were evaluated, in the same manner as Example 1. The evaluation results are shown in Table 1.

### [Comparative Example 2]

With the exception of replacing the ionic compound 1 from Example 1 with a quaternary ammonium ion shown below, ink compositions were prepared, and the curability and charge transport properties were evaluated, in the same manner as Example 1. The evaluation results are shown in Table 1.

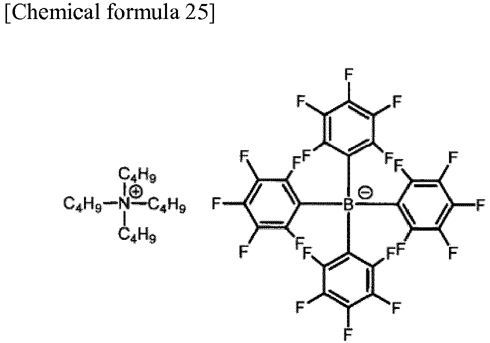

### [Comparative Example 3]

With the exceptions of replacing the charge transport compound 1 from Example 1 with the charge transport compound 2, and replacing the ionic compound 1 with the ionic compound 5, ink compositions were prepared, and the curability and charge transport properties were evaluated, in the same manner as Example 1. The evaluation results are shown in Table 1.

### [Comparative Example 4]

With the exceptions of replacing the charge transport compound 1 from Example 1 with the charge transport compound 2, and replacing the ionic compound 1 with the quaternary ammonium ion used above in Comparative Example 2, ink compositions were prepared, and the curability and charge transport properties were evaluated, in the same manner as Example 1. The evaluation results are shown in Table 1.

### [Comparative Example 5]

With the exceptions of replacing the charge transport compound 1 from Example 1 with the charge transport compound 3, and replacing the ionic compound 1 with the ionic compound 5, ink compositions were prepared, and the curability and charge transport properties were evaluated, in the same manner as Example 1. The evaluation results are shown in Table 1.

### [Comparative Example 6]

With the exceptions of replacing the charge transport compound 1 from Example 1 with the charge transport compound 3, and replacing the ionic compound 1 with the quaternary ammonium ion used above in Comparative Example 2, ink compositions were prepared, and the curability and charge transport properties were evaluated, in the same manner as Example 1. The evaluation results are shown in Table 1.

**[Table 1]**

| | Charge transport compound | HOMO (eV) | Ionic compound | Curability | Charge transport properties |
|---|---|---|---|---|---|
| | | | | Residual film ratio (%) | Applied voltage (V) |
| Example 1 | Charge transport compound 1 | 5.10 | Ionic compound 1 | 98.5 | 2.1 |
| Example 2 | Charge transport compound 1 | 5.10 | Ionic compound 2 | 98.5 | 2.0 |
| Example 3 | Charge transport compound 1 | 5.10 | Ionic compound 3 | 99.6 | 2.2 |
| Example 4 | Charge transport compound 1 | 5.10 | Ionic compound 4 | 97.6 | 1.9 |
| Comparative Example 1 | Charge transport compound 1 | 5.10 | Ionic compound 5 | 99.0 | 3.0 |
| Comparative Example 2 | Charge transport compound 1 | 5.10 | Quaternary ammonium | 20.0 | 10.0 |
| Example 5 | Charge transport compound 2 | 5.35 | Ionic compound 1 | 98.7 | 2.8 |
| Example 6 | Charge transport compound 2 | 5.35 | Ionic compound 2 | 98.2 | 3.1 |
| Example 7 | Charge transport compound 2 | 5.35 | Ionic compound 3 | 99.5 | 3.2 |
| Example 8 | Charge transport compound 2 | 5.35 | Ionic compound 4 | 98.0 | 3.5 |
| Comparative Example 3 | Charge transport compound 2 | 5.35 | Ionic compound 5 | 98.2 | 10.0 |
| Comparative Example 4 | Charge transport compound 2 | 5.35 | Quaternary ammonium | 24.0 | 14.2 |
| Example 9 | Charge transport compound 3 | 5.45 | Ionic compound 1 | 97.5 | 3.6 |
| Example 10 | Charge transport compound 3 | 5.45 | Ionic compound 2 | 98.0 | 3.7 |
| Example 11 | Charge transport compound 3 | 5.45 | Ionic compound 3 | 99.2 | 4.2 |
| Example 12 | Charge transport compound 3 | 5.45 | Ionic compound 4 | 97.0 | 4.3 |
| Comparative Example 5 | Charge transport compound 3 | 5.45 | Ionic compound 5 | 98.0 | 15.0 |
| Comparative Example 6 | Charge transport compound 3 | 5.45 | Quaternary ammonium | 23.0 | 20.5 |

Based on Table 1, it is evident that compared with Comparative Examples 1 and 2, Examples 1 to 4 yielded superior results for both the curability and the charge transport properties at the same time. Similarly, compared with Comparative Examples 3 and 4, or 5 and 6, Examples 5 to 8, and Examples 9 to 12 respectively, exhibited superior results for both the curability and the charge transport properties at the same time.

In other words, it is thought that the organic electronic material according to an embodiment of the present invention exhibits good solvent resistance, facilitates good hole current flow, and also contributes to a reduction in the voltage of the organic electronic element.

### <5> Production and Evaluation of Organic EL Elements

### [Examples 13 to 15, and Comparative Examples 7 to 12]

The charge transport compound 4 (10.0 mg), an ionic compound 6 shown below (0.5 mg) and toluene (2.3 mL) were mixed together under a nitrogen atmosphere to prepare an ink composition for forming a hole injection layer. The ink composition was spin-coated at a rotational rate of 3,000 min⁻¹ onto a glass substrate on which ITO had been patterned with a width of 1.6 mm, thus forming a coating film. The coating film was then cured by heating the substrate on a hot plate at 120°C for 10 minutes, thus forming a hole injection layer (25 nm).

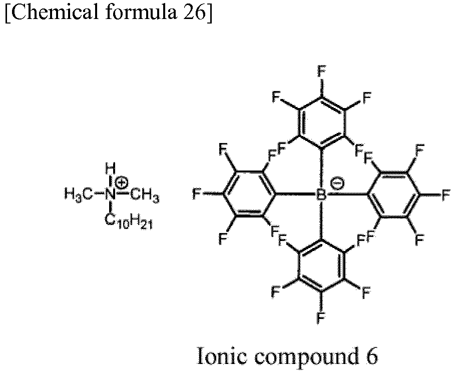

Subsequently, as shown in Table 2, one of the charge transport compounds 1 to 3 (10.0 mg), one of the ionic compound 1, the ionic compound 4 and the quaternary ammonium (0.5 mg), and toluene (1.15 mL) were mixed together to prepare an ink composition for forming a hole transport layer.

The ink composition for forming a hole transport layer was spin-coated at a rotational rate of 3,000 min⁻¹ onto the previously formed hole injection layer, thus forming a coating film. The coating film was then cured by heating on a hot plate at 120°C for 10 minutes, thus forming a hole transport layer (40 nm). In each of the examples and comparative examples, the hole transport layer was able to be formed without dissolving the hole injection layer.

**[Table 2]**

| | Hole injection layer | Hole transport layer |
|---|---|---|
| Example 13 | Charge transport compound 4 Ionic compound 6 | Charge transport compound 1 Ionic compound 1 |
| Example 14 | Charge transport compound 4 Ionic compound 6 | Charge transport compound 2 Ionic compound 1 |
| Example 15 | Charge transport compound 4 Ionic compound 6 | Charge transport compound 3 Ionic compound 1 |
| Comparative Example 7 | Charge transport compound 4 Ionic compound 6 | Charge transport compound 1 Ionic compound 5 |
| Comparative Example 8 | Charge transport compound 4 Ionic compound 6 | Charge transport compound 2 Ionic compound 5 |
| Comparative Example 9 | Charge transport compound 4 Ionic compound 6 | Charge transport compound 3 Ionic compound 5 |
| Comparative Example 10 | Charge transport compound 4 Ionic compound 6 | Charge transport compound 1 Quaternary ammonium |
| Comparative Example 11 | Charge transport compound 4 Ionic compound 6 | Charge transport compound 2 Quaternary ammonium |
| Comparative Example 12 | Charge transport compound 4 Ionic compound 6 | Charge transport compound 3 Quaternary ammonium |

Each of the substrates obtained above, having a sequentially formed hole injection layer and hole transport layer, was transferred into a vacuum deposition apparatus, CBP:Ir(ppy)₃ (94:6, 30 nm), BAlq (10 nm), TPBi (30 nm), LiF (0.8 nm) and Al (100 nm) were deposited in that order by vacuum deposition onto the substrate, and an encapsulation treatment was then performed to complete production of an organic EL element.

When a voltage was applied to the organic EL elements obtained in Examples 13 to 15 and Comparative Examples 7 to 12, green light emission was confirmed in each case. For each element, the emission efficiency at an emission luminance of 1,000 cd/m² and the emission lifespan (luminance half-life) when the initial luminance was 5,000 cd/m² were measured.

The measurement results are shown in Table 3.

**[Table 3]**

| | Drive voltage | Emission efficiency (cd/A) | Emission lifespan (h) |
|---|---|---|---|
| Example 13 | 6.0 | 36.2 | 125.1 |
| Example 14 | 6.4 | 38.2 | 140.3 |
| Example 15 | 6.9 | 39.1 | 154.8 |
| Comparative Example 7 | 6.1 | 32.1 | 100.4 |
| Comparative Example 8 | 8.0 | 30.5 | 55.3 |
| Comparative Example 9 | 9.5 | 29.3 | 40.7 |
| Comparative Example 10 | 8.0 | 13.0 | 10.3 |
| Comparative Example 11 | 10.0 | 12.0 | 7.5 |
| Comparative Example 12 | 13.1 | 10.6 | 11.8 |

Based on a comparison of the above Examples 13 to 15 and Comparative Examples 7 to 12, it is evident that the organic electronic material according to an embodiment of the present invention exhibits superior drive voltage, as well as excellent emission efficiency and lifespan characteristics.

### DESCRIPTION OF THE REFERENCE SIGNS

1: Light-emitting layer
2: Anode
3: Hole injection layer
4: Cathode
5: Electron injection layer
6: Hole transport layer
7: Electron transport layer
8: Substrate

## Claims

1. An organic electronic material comprising at least an ionic compound represented by general formula (1) shown below, and a compound having a charge transport unit: 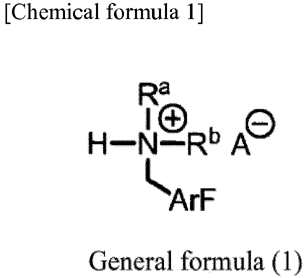 wherein, in general formula (1):
ArF represents a fluoroaryl group or a fluoroheteroaryl group,
each of R^{a} and R^{b} independently represents a hydrogen atom (H), an alkyl group, a benzyl group, an aryl group or a heteroaryl group, and
A represents an anion.

2. The organic electronic material according to Claim 1, wherein the anion is represented by one of general formulas (1b) to (5b) shown below: 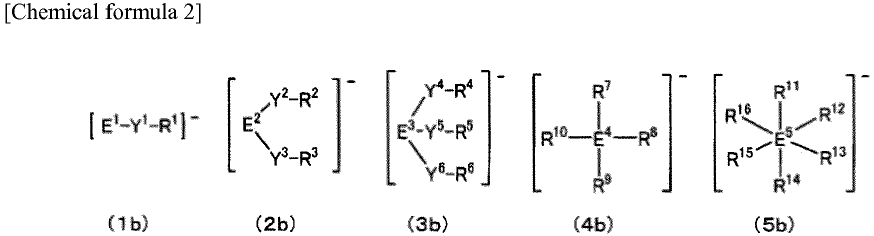 wherein, in general formulas (1b) to (5b):
each of Y¹ to Y⁶ independently represents a single bond or a divalent linking group,
each of R¹ to R¹⁶ independently represents an electron-withdrawing monovalent group, wherein R² and R³, at least two groups selected from among R⁴ to R⁶, at least two groups selected from among R⁷ to R¹⁰, and at least two groups selected from among R¹¹ to R¹⁶, may each be bonded together to form a ring, and
E¹ represents an oxygen atom, E² represents a nitrogen atom, E³ represents a carbon atom, E⁴ represents a boron atom or a gallium atom, and E⁵ represents a phosphorus atom or an antimony atom.

3. The organic electronic material according to Claim 1 or 2, wherein at least one of R^{a} and R^{b} is an alkyl group, a benzyl group, an aryl group or a heteroaryl group.

4. The organic electronic material according to Claim 3, wherein at least one of R^{a} and R^{b} is an alkyl group or a benzyl group.

5. The organic electronic material according to any one of Claims 1 to 4, wherein ArF is a fluoroaryl group.

6. The organic electronic material according to any one of Claims 1 to 5, wherein the charge transport unit is an aromatic amine, a carbazole or a thiophene.

7. The organic electronic material according to any one of Claims 1 to 6, wherein the compound having a charge transport unit is a polymer or an oligomer.

8. The organic electronic material according to any one of Claims 1 to 7, wherein the compound having a charge transport unit has at least one polymerizable functional group.

9. The organic electronic material according to Claim 8, wherein the polymerizable functional group is at least one group selected from the group consisting of an oxetanyl group, an epoxy group and a vinyl ether group.

10. An ink composition comprising the organic electronic material according to any one of Claims 1 to 9, and a solvent.

11. An organic electronic element comprising an organic layer formed using the organic electronic material according to any one of Claims 1 to 9, or the ink composition according to Claim 10.

12. The organic electronic element according to Claim 11, wherein the organic electronic element comprises multilayered organic layers produced by forming a separate organic layer on top of the organic layer.

13. The organic electronic element according to Claim 12, wherein at least one of the organic layer and the separate organic layer is at least one layer selected from the group consisting of a hole injection layer, a hole transport layer and a light-emitting layer.

14. The organic electronic element according to any one of Claims 11 to 13,
further comprising a substrate, wherein
the substrate is a resin film.

15. The organic electronic element according to any one of Claims 11 to 14, wherein the organic electronic element is an organic electroluminescent element.

16. A method for producing an organic electronic element that comprises a step of forming an organic layer by a coating method using the organic electronic material according to any one of Claims 1 to 9 or the ink composition according to Claim 10.

17. The method for producing an organic electronic element according to Claim 16, further comprising a step of polymerizing and insolubilizing the organic layer formed by a coating method.

18. The method for producing an organic electronic element according to Claim 17, further comprising a step of performing multilayering by forming a separate organic layer on top of the insolubilized organic layer.
